# EUROPEAN PATENT SPECIFICATION

(11) **EP 1 943 516 B1**
(45) Date of publication and mention of the grant of the patent: **07.01.2015**
(21) Application number: 06827486.9
(22) Date of filing: 02.11.2006
(51) Int. Cl.: G01N 33/50, G01N 33/574

(54) **COMPOSITE PROFILES OF CELL ANTIGENS AND TARGET SIGNAL TRANSDUCTION PROTEINS FOR ANALYSIS AND CLINICAL MANAGEMENT OF HEMATOLOGIC CANCERS**
VERBUNDPROFILE VON ZELLANTIGENEN UND ZIELSIGNALTRANSDUKTIONSPROTEINEN ZUR ANALYSE UND KLINISCHEN BEHANDLUNG HÄMATOLOGISCHER KREBSERKRANKUNGEN
PROFILES COMPOSITES D'ANTIGÈNES CELLULAIRES ET DE PROTÉINES DE TRANSDUCTION DE SIGNAL CIBLE DESTINÉS À L'ANALYSE ET AU TRAITEMENT CLINIQUE DE CANCERS HÉMATOLOGIQUES

(30) Priority: 04.11.2005 US 267948
(43) Date of publication of application: 16.07.2008
(73) Proprietor: Beckman Coulter, Inc., Brea, CA 92821 (US); Northwestern University, Evanston, IL 60208 (US); University Health Network, Toronto, Ontario M5G 2M9 (CA); CASE WESTERN RESERVE UNIVERSITY, Cleveland, Ohio 44106 (US); ALLEGHENY-SINGER RESEARCH INSTITUTE, Pittsburgh, PA 15212 (US)
(72) Inventor: GOOLSBY, Charles, Winfield, IL 60190 (US); SHANKEY, Vincent, T., Miami, FL 33187 (US); HEDLEY, David, Toronto, ON M4Y 2W1 (CA); JACOBBERGER, James, Chesterland, OH 44026 (US); SHACKNEY, Stanley, Pittsburgh, PA 15243 (US)
(74) Representative: Boult Wade Tennant
(86) International application number: PCT/US2006/043050
(87) International publication number: WO 2007/056192

(56) References cited:
- EP-A- 1 777 523
- US-A1- 2003 148 321
- US-A1- 2003 203 416
- WAGNER SIMON D ET AL: "Chronic lymphocytic leukaemia: new biological markers for assessing prognosis" HEMATOLOGY JOURNAL, MCMILLAN, BASINGSTOKE, GB, vol. 5, no. 3, 1 January 2004 (2004-01-01), pages 197-201, XP009118389 ISSN: 1466-4860
- ZOLTAN MATRAI: "CD38 as a prognostic marker in CLL" HEMATOLOGY, vol. 10, no. 1, 1 February 2005 (2005-02-01), pages 39-46, XP009121406
- DI BACCO A. ET AL.: 'Molecular Abnormalities in Chronic Myeloid Leukemia: Deregulation of Cell Growth and Apoptosis' THE ONCOLOGIST vol. 5, no. 5, October 2000, pages 405 - 417, XP003015406
- LIU ET AL.: 'Overexpression of Cyclin D1 in Accelerated-Phase Chronic Myeloid Leukemia' LEUKEMIA & LYMPHOMA vol. 45, no. 12, December 2004, pages 2419 - 2425, XP008082617
- BAGRINTSEVA K. ET AL.: 'FLT3-ITD-TKD dual mutants associated with AML confer resistance to FLT3 PTK inhibitors and cytotoxic agents by overexpression of Bcl-x(L)' BLOOD vol. 105, no. 9, May 2005, pages 3679 - 3685, XP003015407
- NEBEN K. ET AL.: 'Gene expression patterns in acute myeloid leukemia correlate with centrosome aberrations and numerical chromosome changes' ONCOGENE vol. 23, no. 13, March 2004, pages 2379 - 2384, XP009031106

## Description

### BACKGROUND OF THE INVENTION

Leukemia is a malignant cancer of the bone marrow and blood. Leukemia is characterized by an excessive production of abnormal white blood cells, overcrowding the bone marrow and/or peripheral blood. This results in decreased production and function of normal blood cells. Chronic Lymphocytic Leukemia (CLL) is one of the four major types of leukemia encountered by humans, the others being Acute Myelogenous Leukemia (AML), Chronic Myelogenous Leukemia (CML), and Acute Lymphocytic Leukemia (ALL).

Leukemia patients follow heterogeneous clinical courses. Treatment of leukemia is very complex and depends upon the type of leukemia. Tremendous clinical variability among remissions is also observed in leukemic patients, even those that occur after one course of therapy. Some survive for prolonged periods without definitive therapy, while others die rapidly despite aggressive treatment. Patients who are resistant to therapy have very short survival times, regardless of when the resistance occurs. While various staging systems have been developed to address this clinical heterogeneity, they cannot accurately predict whether an early or intermediate stage patient will experience an indolent or aggressive course of disease. Specifically, since these systems consider gross manifestations of the disease, including the level of blood and marrow lymphocyte counts, the size and distribution of the lymph nodes, the spleen size, the degree of anemia and the patient's blood platelet count, they can only identify patients with poor prognostic outcome when the disease has progressed to a more advanced state.

Chronic Lymphocytic Leukemia (CLL) is the most common leukemia diagnosed in the US and Europe with over 10,000 new cases in the United States in 2004, and in most cases, presents in men over 40 years of age. It is characterized by the clonal expansion of small lymphocytes, most commonly demonstrating surface markers CD5 and CD19 consistent with a subset of B-lymphocytes. In the majority of cases, the disease is indolent, with patients surviving for prolonged periods without definitive therapy. In addition, gender is relevant, since men outnumber women by an approximate 2:1 ratio. The minority of cases, frequently in younger individuals, the disease rapidly progresses despite aggressive treatments. In the more common, more indolent form, it has a gradual onset, and may not cause the patient discomfort or pain for several years.

CLL is characterized by a large number of cancerous mature lymphocytes and enlarged lymph nodes. Cancerous cells crowd out the normal cells in the bone marrow and lymph nodes. Anemia develops in the patient and the number of normal white cells and platelets in the patient's blood decreases, whereas the total white cell count increases due to the proliferation of abnormal white cells. The level and activity of antibodies also decrease. As a result, the patient's immune system becomes compromised. It is more common for CLL sufferers to die from consequences of the compromised immune system such as infections, than from the CLL itself.

Clinical stage of CLL, characterized in the staging systems of Rai (stages O-IV) and Binet (stages A-C), remains the strongest predictor of survival in CLL patients. Both system are based on the amount of involved lymphoid tissue and the presence of anemia and/or thrombocytopenia. In general, patients with later stages have a significantly worse prognosis and a shorter survival. Patients with Rai stage IV or Binet stage C have a median survival of only 1.5 to 2 years.

Knezevic et al., Proteomics 2001; Vol 1, pages 1271-1278 describes a study involving high-throughput proteomic analysis of frozen tissue sections derived from squamous cell carcinoma of the oral cavity.

Wagner et al., Hematology Journal 2004; Vol. 5, No. 3, pages 197-201 reviews a range of prognostic markers for deciding when to initiate treatment and assigning prognosis in Chronic Lymphocytic Leukaemia (CLL) and the various techniques available for measuring these markers.

EP1777523 relates to an *in vitro* method for the prognosis of patients for progression of cancer. EP1777523 was published on 25 April 2007, after the filling date of the present application. It is therefore only citable as prior art for novelty purposes under Article 54(3) EPC.

Zoltan Matrai, Hematology 2005, Vol. 10. No. 1, pages 39-46 reviews the use of cell surface expression of CD38 as a marker of progressive disease and poor outcome in Chronic Lymphocytic Leukaemia (CLL).

US 2003/148321 describes methods and kits for distinguishing between heparanase expressing and heparanase non-expressing hematopoietic cells.

At the present time, there is no known treatment for B-CLL which has been shown to definitively increase life expectancy. Consequently, only patients classified in the advanced stages of B-CLL have been considered for aggressive treatment such as chemotherapy, radiation therapy, surgery, immunotherapy or transplantation. These treatments may exact a severe physical and emotional toll on the patient without necessarily improving outcome; in some instances, B-CLL patients may even succumb from the rigors of treatment rather than from the effects of B-CLL. Patients classified in the early stages of B-CLL, who may be in better physical condition to receive more aggressive or experimental treatment, generally receive no treatment as long as the condition remains stable for two reasons. First, currently available therapies do not extend life span. Second, there are currently no reliable indicators of which early stage patients will do well and which will do poorly. Further, the unpredictable course of the disease can make interpreting the results of clinical trials difficult, as some early stage patients will follow an indolent course even without the benefit of treatment.

In view of the heterogeneous clinical courses and tremendous clinical variability among remissions among leukemic patients, there is a need for a reliable indicator of an individuals predicted disease course to help clinicians identify those patients that progress to a more advanced state of the disease and allow the option of more aggressive or experimental treatment at a much earlier stage. Additionally, clinical trials of new drugs or experimental therapies could be directed to patients depending upon their prognostic outlook, thereby allowing for more relevant results in clinical trials.

The present invention satisfies this need and provides related advantages as well.

### SUMMARY OF THE INVENTION

The present invention is directed to methods for establishing a composite marker profile for a sample derived from an individual suspected having a neoplastic condition. A composite marker profile of the invention allows for identification of prognostically and therapeutically relevant subgroups of neoplastic conditions and prediction of the clinical course of an individual. The methods of the invention provide tools useful in choosing a therapy for an individual afflicted with a neoplastic condition, including methods for assigning a risk group, methods of predicting an increased risk of relapse, methods of predicting an increased risk of developing secondary complications, methods of choosing a therapy for an individual, methods of predicting response to a therapy for an individual, methods of determining the efficacy of a therapy in an individual, and methods of determining the prognosis for an individual. In particular, the method of the present invention discloses a method for establishing a composite marker profile that can serve as a prognostic indicator to predict whether the course of a neoplastic condition in a individual will be aggressive or indolent, thereby aiding the clinician in managing the patient and evaluating the modality of treatment to be used. The invention is defined in the claims.

In particular embodiments disclosed herein, the methods of the invention are directed to establishing a composite marker profile for a leukemia selected from the group consisting of Chronic Lymphocytic Leukemia (CLL), Acute Myelogenous Leukemia (AML), Chronic Myelogenous Leukemia (CML), and Acute Lymphocytic Leukemia (ALL). A composite marker profile established by the disclosed methods can be useful for all aspects of clinical management of an individual afflicted with leukemia, including, for example, assigning a individual afflicted with leukemia to a leukemia risk group, predicting whether a individual afflicted with leukemia has an increased risk of relapse, predicting whether a individual afflicted with by leukemia has an increased risk of developing a secondary leukemia; methods to aid in the determination of a prognosis for a individual afflicted with leukemia, methods of choosing a therapy for a individual afflicted with leukemia, and methods of monitoring the disease state in a individual undergoing one or more therapies for leukemia.

### BRIEF DESCRIPTION OF THE DRAWINGS

Figure 1 shows a schematic diagram that exemplifies sample preparation for methods for establishing a composite marker profile.
Figure 2 shows flow cytometry panels obtained from a gating strategy protocol for ZAP-70 analysis in a CLL sample.
Figure 3 shows flow cytometry panels obtained from a gating strategy for ZAP-70 analysis in CLL sample.
Figure 4 shows a diagram depicting ZAP-70 expression in the universe of cells present in a CLL sample. (red: B-cells; green: normal T-cells; purple: NK cells)
Figure 5 shows flow cytometry panels depicting S6 activation by mTOR in a AML sample.
Figure 6 shows a Composite Profile of Mobilized Stem Cells (CD34+) in Peripheral Blood. The histograms provide a comparison of the responses of of three different signal transduction proteins, P-ERK, P-S6, and P-PKB/AKT in each of mobilized stem cells, normal granulocytes, normal monocytes and normal lymphocytes after stimulation with PMA (blue), Stem Cell Factor (green), or IGF-1 (orange), compared to untreated sample (red).
Figure 7 shows a composite profile of a single AML Patient. Blood aliquots are stimulated with PMA, with or without specific signal transduction pathway inhibitors ("map" in lower right corner), or with Stem Cell Factor. The responses are measured as bivariate plots of P-ERK versus P-S6 (right panels), where the red population is the AML blasts, the blue the internal lymphocytes, and the green, the internal granulocytes. The AML blasts (red), Lymphocytes (blue) and Granulocytes (green) are identified using CD34 versus SSC as depicted in the two boxes on the left.
Figure 8 shows composite profiles ZAP-70 Expression in CLL. Green: CD5negB-cells; Blue: CD5+ B-cells; Pink: T-cells (CD5+/CD3+); Purple: NK-cells (CD5neg/CD56+). Overlayed histograms are shown with each histogram separately autoscaled to the number of events for that subset.

### DETAILED DESCRIPTION OF THE INVENTION

This invention is directed to methods for establishing a composite marker profile for a sample derived from an individual suspected having a neoplastic condition. A composite marker profile of the invention allows for identification of prognostically and therapeutically relevant subgroups of neoplastic conditions and prediction of the clinical course of an individual. The methods of the invention provide tools useful for clinical management of an individual afflicted with a neoplastic condition, including methods for assigning a risk group, methods of predicting an increased risk of relapse, methods of predicting an increased risk of developing secondary complications, methods of choosing a therapy for an individual, methods of determining the efficacy of a therapy in an individual, and methods of determining the prognosis for an individual.

The invention is based, in part, on the discovery that, by correlating the presence of distinct cell population associated markers with one or more selected target proteins, it is possible to prepare a composite marker profile that represents a quantitative measurement based on comparison of internal cell populations across the sample positive and negative for the measurement of interest.

In particular, the method of the present invention discloses a method for establishing a composite marker profile that can serve as a prognostic indicator to predict whether the clinical course of a neoplastic condition in a individual will be aggressive or indolent, thereby aiding the clinician in managing the patient and evaluating the modality of treatment to be used.

In particular embodiments disclosed herein, the methods of the invention are directed to establishing a composite marker profile for a leukemia selected from the group consisting of Chronic Lymphocytic Leukemia (CLL), Acute Myelogenous Leukemia (AML), Chronic Myelogenous Leukemia (CML), and Acute Lymphocytic Leukemia (ALL). A composite marker profile established by the disclosed methods can be useful for all aspects of clinical management of an individual afflicted with leukemia, including, for example, assigning a individual afflicted with leukemia to a leukemia risk group, predicting whether a individual afflicted with leukemia has an increased risk of relapse, predicting whether a individual afflicted with by leukemia has an increased risk of developing a secondary leukemia; methods to aid in the determination of a prognosis for a individual afflicted with leukemia, methods of choosing a therapy for a individual afflicted with leukemia, and methods of monitoring the disease state in a individual undergoing one or more therapies for leukemia.

A composite profile established by the methods of the invention encompasses reacting the biological sample with a collection of binding molecules, wherein the collection of binding molecules contains two or more groups of binding molecules specific for distinct cell population associated markers. The marker levels and marker combinations identify normal and neoplastic cell populations internal to the sample and are used to identify the presence of normal and neoplastic cell populations internal to the sample by detecting the marker levels and marker combinations that identify normal and neoplastic cell populations internal to the sample. Finally, by correlating the marker levels across each of the normal and neoplastic cell populations with the presence of a target protein, the method of the invention allows for establishment of a composite marker profile for a sample derived from an individual suspected having a neoplastic condition. The target protein is a signaling molecule.

A composite profile established by a method of the invention has unexpected advantages and overcomes various problems encountered in the art of predicting disease course based on the presence of cellular markers in a biological sample. As described herein, a target protein of the invention allows for determination of the semi-quantitative levels of a cellular target of interest based on reference to multiple cell populations that are contained in the sample and known to be negative or positive for the cellular target of interest. In the methods described herein, the presence and level of the cellular target of interest is correlated separately to multiple reference positive populations that are contained in the sample and that are characterized by variable levels of the cellular target of interest. In addition, the method correlates the presence and level of the cellular target of interest to one or more internal cell populations within the sample that are known to be negative for the target protein. Thus, rather than using an unrelated antigen to provide an internal standard for quantification, the methods of the invention correlate multiple internal populations that are positive for the cellular target of interest and allow for the development of targeted therapeutic strategies based on an individual's composite profile. The methods provided by the invention can be generalized to quantification of any cellular targets for which there are reference negative and positive populations within the sample.

In one embodiment, the invention provides a method for establishing a composite marker profile for a sample derived from an individual suspected of having a neoplastic condition that includes the steps of reacting the biological sample with a collection of binding molecules, wherein the collection of binding molecules contains two or more groups of binding molecules specific for distinct cell population associated markers, and wherein marker levels and marker combinations identify normal and neoplastic cell populations internal to the sample, identifying the presence of normal and neoplastic cell populations internal to the sample by detecting the marker levels and marker combinations that identify the normal and neoplastic cell populations internal to the sample, and subsequently correlating the marker levels across each of the normal and neoplastic cell populations with the presence of a target protein to establish a composite marker profile, wherein the target protein is a signaling molecule.

In particular embodiments, the methods of the invention are applied to a cancer that is further defined as a "hematologic cancer," a term that refers to malignant neoplasms of blood-forming tissues and encompasses leukemia, lymphoma and multiple myeloma. Leukemias include acute myelogenous leukemia or acute myeloid leukemia (AML), chronic myelogenous leukemia or chronic myeloid leukemia (CML), chronic lymphocytic leukemia (CLL), acute lymphocytic leukemia (ALL), hairy cell leukemia (HCL), myelodysplastic syndromes (MDS) or chronic myelogenous leukemia (CML-BP) in blastic and all subtypes of these leukemias which are defined by morphological, histochemical and immunological techniques that are well known by those of skill in the art. In particular embodiments of the invention, the hematologic cancer is a selected from the group consisting of Chronic Lymphocytic Leukemia (CLL), Acute Myelogenous Leukemia (AML), Chronic Myelogenous Leukemia (CML), and Acute Lymphocytic Leukemia (ALL).

Leukemias were originally divided into acute or chronic leukemias based on life expectancy but now are classified according to cellular maturity. Acute leukemias consist of predominantly immature cells (usually blast forms); chronic leukemias consist of more mature cells. Acute leukemias are characterized by the rapid growth of immature blood cells. This crowding makes the bone marrow unable to produce healthy blood cells. Acute leukemias are further divided into lymphoblastic (ALL) and myelogenous (AML) types, which can be further subdivided by morphologic and cytochemical appearance according to the French-American-British (FAB) classification or immunophenotype. The specific B-cell and T-cell and myeloid-antigen monoclonal antibodies, together with flow cytometry, are very helpful for classifying ALL versus AML, which is critical for treatment.

The acute leukemias consist of acute lymphoblastic leukemia (ALL) and acute myelogenous leukemia (AML). Leukemic cells accumulate in the bone marrow, replace normal hematopoietic cells, and spread to the liver, spleen, lymph nodes, CNS, kidneys, and gonads. Because the cells are bloodborne, they can infiltrate any organ or site. ALL often involves the CNS, whereas acute monoblastic leukemia involves the gums, and AML involves localized collections in any site (granulocytic sarcomas or chloromas).

Chronic leukemias are described as lymphocytic (CLL) or myelocytic (CML). CLL involves clonal expansion of mature-appearing lymphocytes involving lymph nodes and other lymphoid tissues with progressive infiltration of bone marrow and presence in the peripheral blood. Traditional delineation of CLL has been of the most common subtype, in particular the B-cell form, which represents almost all cases. In 2% to 3% of cases, the clonal expansion is T cell in type, and even this group has a subtype, in particular, large granular lymphocytes with cytopenias. In addition, other chronic leukemic patterns have been categorized under CLL: prolymphocytic leukemia, leukemic phase of cutaneous T-cell lymphoma (Sézary syndrome), hairy cell leukemia, and lymphoma leukemia (leukemic changes seen in advanced stages of malignant lymphoma). Differentiation of these subtypes from typical CLL is usually straightforward.

In one embodiment, the invention provides a method for establishing a composite marker profile for a sample derived from an individual suspected having B-Cell Chronic Lymphocytic Leukemia (B-CLL) by reacting the biological sample with a collection of binding molecules containing two or more groups of binding molecules specific for distinct cell population associated markers and identifying the presence of normal and neoplastic cell populations internal to the sample by detecting the marker levels and marker combinations that identify the normal and neoplastic cell populations internal to the sample, and correlating the marker levels across each of the normal and neoplastic cell populations with the presence of ZAP-70 to establish a composite marker profile for a sample derived from an individual suspected having B-Cell Chronic Lymphocytic Leukemia (B-CLL).

B-CLL is characterized by the clonal accumulation of CD5⁺ cells (Caligaris-Cappio, et al., J Exp Med 155:623-8, 1982). The expression of specific cell surface markers distinguishes subsets of normal human B cells that differ in differentiation and activation stages and in biologic properties (Clark and Lane, Ann Rev Immunol 9:97-127, 1991). In particular, analyses of CD38 and IgD expression have been especially useful in distinguishing B-cells at various stages of differentiation from naive through memory cells (Pascual, et al., J Exp Med 180:329-339, 1994; Zupo, et al., Blood, 88:1365-1374, 1996).

In a further embodiment, the present invention provides a method for establishing a composite marker profile for a sample derived from an individual suspected having CML by reacting the biological sample with a collection of binding molecules that contains two or more groups of binding molecules specific for distinct cell population associated markers, wherein marker levels and marker combinations identify normal and neoplastic cell populations internal to the sample; subsequently identifying the presence of normal and neoplastic cell populations internal to the sample by detecting the marker levels and marker combinations that identify the normal and neoplastic cell populations internal to the sample, and correlating the marker levels across each of the normal and neoplastic cell populations with the presence of at least one target protein to establish a composite marker profile for a sample derived from an individual suspected having Chronic Myelogenous Leukemia (CML). In this embodiment, the target protein can be selected from the group consisting of activation-phosphorylated signal transduction proteins, proliferation markers, differentiation markers and apoptosis markers.

Chronic myelogenous leukemia (CML) is a disease having clinical and pathological features distinct from those of other forms of leukemia. It is widely accepted that the cause of CML is a specific chromosomal translocation between human chromosome 9 and human chromosome 22. The abnormal chromosome resulting from this translocation is commonly referred to as the Philadelphia chromosome (Darnell, J. et al., Molecular Cell Biology, 2nd Ed., W. H. Freeman and Co., New York (1990), p. 992). The gene for c-abl (ABL), a tyrosine kinase thought to be involved in growth control, resides on the distal arm of human chromosome 9, while the gene for c-bcr (BCR) resides on human chromosome 22. The translocation places the promoter distal three exons of ABL, including those elements which encode the tyrosine kinase domain, downstream of either the first or second exon of BCR. Chung, S. and Wong, P. M. C., Oncogene, 10:1261-1268 (1995). The product of the translocation between human chromosome 9 and human chromosome 22 is a chimeric gene, BCR-ABL, which encodes a fusion protein, often referred to as p185^{bcr-abl} or p210^{bcr-abl}, depending upon the inclusion of the second exon of BCR. Bartram, C. R., et al., Nature, 306:277-280 (1983). p185^{bcr-abl} causes acute leukemia, typically lymphoblastic; p210^{bcr-abl} usually causes CML, but can occasionally also cause acute leukemia.

CML involves clonal myeloproliferation caused by malignant transformation of a pluripotent stem cell and is characterized clinically by excessive production of granulocytes, primarily in the bone marrow but also in extramedullary sites (eg, spleen, liver). Although granulocyte production predominates, the neoplastic clone includes RBC, megakaryocyte, monocyte, and even some T and B cells. Normal stem cells are retained and can emerge after drug suppression of the CML clone. The bone marrow is hypercellular, but in 20 to 30% of patients, myelofibrosis develops, usually after several years. In most patients, the CML clone progresses to an accelerated phase and final blast crisis. At this time, blast cell tumors can develop in other extramedullary sites including, bone, CNS, lymph nodes, and skin.

Chronic myelogenous leukemia (CML) exhibits a characteristic disease course, presenting initially as a chronic granulocytic hyperplasia, and invariably evolving into an acute leukemia which is caused by the clonal expansion of a cell with a less differentiated phenotype (i.e., the blast crisis stage of the disease). CML is an unstable disease which ultimately progresses to a terminal stage which resembles acute leukemia. This lethal disease affected approximately 9,730 patients in the United States in 2004. Chemotherapeutic agents such as hydroxyurea or busulfan can reduce the leukemic burden but do not impact the life expectancy of the patient (e.g. approximately 4 years). Consequently, CML patients are candidates for bone marrow transplantation (BMT) therapy. However, for those patients which survive BMT, disease recurrence remains a major obstacle (Apperley et al., 1988 Br. J. Haematol. 69, 239).

In an additional embodiment, the present invention provides a method for establishing a composite marker profile for a sample derived from an individual suspected having AML by reacting the biological sample with a collection of binding molecules that contains two or more groups of binding molecules specific for distinct cell population associated markers, wherein marker levels and marker combinations identify normal and neoplastic cell populations internal to the sample; subsequently identifying the presence of normal and neoplastic cell populations internal to the sample by detecting the marker levels and marker combinations that identify the normal and neoplastic cell populations internal to the sample, and correlating the marker levels across each of the normal and neoplastic cell populations with the presence of at least one target protein to establish a composite marker profile for a sample derived from an individual suspected having Acute Myelogenous Leukemia (AML). In this embodiment, the target protein can be selected from the group consisting of signal transduction proteins, proliferation markers and apoptosis markers.

In a further embodiment, the present invention provides a method for establishing a composite marker profile for a sample derived from an individual suspected having Acute Lymphocytic Leukemia (ALL) by reacting the biological sample with a collection of binding molecules that contains two or more groups of binding molecules specific for distinct cell population associated markers, wherein marker levels and marker combinations identify normal and neoplastic cell populations internal to the sample; subsequently identifying the presence of normal and neoplastic cell populations internal to the sample by detecting the marker levels and marker combinations that identify the normal and neoplastic cell populations internal to the sample, and correlating the marker levels across each of the normal and neoplastic cell populations with the presence of at least one target protein to establish a composite marker profile for a sample derived from an individual suspected having ALL. In this embodiment, the target protein can be selected from the group consisting of signal transduction proteins, proliferation markers and apoptosis markers.

As used herein, the term "neoplastic condition" refers to a condition associated with proliferation of cells characterized by a loss of normal controls that results in one ore more symptoms including, unregulated growth, lack of differentiation, local tissue invasion, and metastasis. As described herein, when establishing a composite marker profile for a sample derived from an individual suspected having a neoplastic condition a biological sample is contacted with a collection of binding molecules that contains two or more groups of binding molecules specific for distinct cell population associated markers that identify normal and neoplastic cell populations internal to the sample.

The biological sample is preferably collected directly from the individual, particularly a human suspected of having cancer and subsequently provided in a form appropriate for establishing a composite profile. In one embodiment, the biological fluid can be, for example, whole blood, peripheral blood mononuclear cells (PBMCs), bone marrow aspirate, or lymphoid tissue. Appropriate biological samples include any body tissue or body fluid. In preferred embodiments, the body tissue can be bone marrow aspirate, bone marrow biopsy, lymph node aspirate, lymph node biopsy, spleen tissue, fine needle aspirate, skin biopsy or organ tissue biopsy, tissue sections and desegregated cells. Other embodiments include samples where the body fluid is peripheral blood, lymph fluid, ascites, serous fluid and cerebrospinal fluid. For acute leukemias, whole blood or bone marrow aspirate are particularly appropriate biological samples. In embodiments where the biological sample comprises whole blood, providing the sample can include fixing and permeabilizing of white blood cells and lysis of red blood cells. It is further understood that providing a biological sample containing significant levels of red blood cells can involve treatment to remove red blood cells, for example, by hypotonic lysis, detergent treatment, and density-gradient centrifugation.

As described herein, in a method of the invention, the biological sample is contacted with a collection of binding molecules that contains two or more groups of binding molecules specific for distinct cell population associated markers that identify normal and neoplastic cell populations internal to the sample. As used herein, the term "binding molecule" refers to any molecule capable of binding to an antigen with sufficient affinity to demonstrate selective binding activity. Examples of binding molecules include antibodies, fragments of antibodies or antibody-like molecules. Antibodies can be produced by B-cells or hybridomas and chimeric or humanized antibodies or any fragment thereof, e.g. F(ab')₂ and Fab fragments, as well as single chain or single domain antibodies.

A binding molecule useful in practicing the methods of the invention can be single chain antibody consists of the variable domains of an antibody heavy and light chains covalently bound by a peptide linker usually consisting of from 10 to 30 amino acids, preferably from 15 to 25 amino acids. Therefore, such a structure does not include the constant part of the heavy and light chains and it is believed that the small peptide spacer should be less antigenic than a whole constant part. A binding molecule useful in practicing the methods of the invention also can be a chimeric antibody, which means an antibody in which the constant regions of heavy or light chains or both are of human origin while the variable domains of both heavy and light chains are of non-human, for example, murine origin. A binding molecule useful in practicing the methods of the invention also can be a humanized antibody that in which the hypervariable regions (CDRs) are of non-human, for example, murine origin, while all or substantially all the other parts of the immunoglobulin, in particular, the constant regions and the highly conserved parts of the variable domains, in particular, the framework regions, are of human origin. A humanized antibody can retain a few amino acids of the murine sequence in the parts of the framework regions adjacent to the hypervariable regions. Hypervariable regions can be associated with any kind of framework regions, preferably of murine or human origin. Suitable framework regions are well known and described in the art. All of the aforementioned binding molecules are useful for practicing the methods of the invention.

A collection of binding molecules as used in a method of the invention contains two or more groups of binding molecules specific for distinct cell population associated markers that identify normal and neoplastic cell populations internal to the sample. As used herein, a "cell population associated marker" refers to an antigen that, alone or in combination with one or more distinct antigens, is used to identify one or more cell populations internal to a biological sample by virtue of being selectively bound by a binding molecule. As is understood in the art, cell populations can be distinguished based on unique combinations of markers, for example, cell surface markers, that are displayed on the cell surface. For acute leukemias, cell population associated markers can include, for example, CD45, CD2, CD3, CD7, CD10, CD11b, CD13, CD14, CD15, CD19, CD20, CD33, CD34, CD56, CD71, CD117, HLA-DR. Supplemental markers can be added as determined by the user, for example, CD1a, cCD3, CD4, CD5, CD8, cCD22, CD61, glycophorin A, and TdT. As described herein, for a biological sample of an individual suspected of having AML, the cell population associated markers useful for establishing a composite profile can include, for example, CD45, CD33, CD34, CD11b, CD13, CD14, CD15, CD16, MPL (myeloperoxidase), CD64, CD117 (c-kit receptor). In addition, for M6-M7 category leukemias markers can be added as determined by the user, for example, CD41, CD42, CD61, CD62P, CD71, GlycophorinA, hemoglobin. It is understood that fewer markers will be analyzed when specimen cellularity is limited. CD molecules are convenient diagnostic markers for identifying and quantitating cell populations by flow cytometry. Flow cytometry is a technique for counting, examining and sorting microscopic particles suspended in a stream of fluid. Flow cytometric procedures start with procurement of cells or tissues, proceed through staining and washing, continue through acquisition of flow data on the cytometer, and culminate in data analysis and the reporting of results.

Cell populations of interest for practicing the methods described herein include various types of white blood cells, in particular granulocytes, lymphocytes and monocytes. There are three types of granulocytes: neutrophils, basophils and eosinophils. White blood cells further include lymphocytes, which encompass B-cells, T-cells and Natural Killer Cells. Natural killer cells or "NK cells" are large granular lymphocytes comprising 2-15% of peripheral blood mononuclear cells in healthy individuals. Although most NK cells are CD3:TCR⁻, CD16⁺, CD56⁺, there is considerable phenotypic and functional heterogeneity within this population (Trinchieri, Adv. Immunol., 47:187 (1989)). For example, the surface density of CD56 has been shown to define functionally distinct NK cell populations. CD56^{bright} NK cells are largely CD16⁺, agranular lymphocytes deficient in cytolytic effector function that proliferate vigorously in response to exogenous IL-2. CD56^{dim} NK cells are CD16⁺LGLs possessing potent cytolytic effector function that do not proliferate in response to IL-2. Because some T cells express both CD16 and CD56, these molecules, by themselves, cannot define the NK cell population. (Trinchieri, 1989). Furthermore, because the expression of CD56 on the functionally differentiated population ofNK cells is low, monoclonal antibodies reactive with CD56 cannot be used to reliably distinguish this subpopulation ofNK cells from other cells in a sample. In addition to being able to distinguish between normal cell populations, binding molecules are used in a method of the invention to identify neoplastic cell populations internal to the sample. CD molecules are convenient diagnostic markers for identifying and quantitating cell populations by flow cytometry. In preferred embodiments of the invention, the level of cell population associated marker expression, for example CD38⁺ for B-CLL expression, is determined using flow cytometry where the cells have been labeled with monoclonal antibodies conjugated with fluorescent dyes or enzymes, although visual immunofluorescence or other methods may also be used. In a specific embodiments, biological samples are analyzed for surface expression of combinations of cell population associated markers, for example, by multiple-color immunofluorescence using a collection of binding molecules specific to a particular cell type associated population. It is understood that any combination of binding molecules can be selected that are specific to identify particular cell type associated population in a biological sample.

As described herein, for a biological sample of an individual suspected of having CLL, the cell population associated markers useful for establishing a composite profile can include, for example, CD3 to identify T-cells, CD5 to identify normal T-cells and malignant B-cells, CD19 to identify normal B-cells and malignant B-cells, CD56 to identify normal NK cells, CD23 to identify activated B-cells or follicular mantle-zone B-cells, CD38 to identify cell activation and or differentiation, CD79b as a cytoplasmic B-cell linage marker, and FMC7 as a normal B-cell lineage marker that is characteristically negative in B-CLL. Thus, marker combinations useful for identification of cell populations in B-CLL include CD5 positive and CD19 positive; CD5 positive and CD20 negative; CD3 positive or CD56 positive; and CD3 positive.

The method may be performed using any tissue containing B-CLL cells, including but not limited to spleen, lymph nodes, bone marrow, lymph, peripheral blood, a whole blood sample from the individual or a whole blood sample that has been treated and processed to isolate the peripheral blood mononuclear cells ("PBMC").

The result of a neoplastic transformation of an early hematopoietic precursor cell, chronic myelogenous leukemia (CML) is characterized clinically by the accumulation of immature and mature myeloid cells in the peripheral circulation and cytogenetically by the presence of the Philadelphia chromosome. The myeloid antigens CD33, CD13 and CD11c, CD45 and the isoform CD45RO are expressed in CML, but not CD45RA which helps to differentiate CML from acute myeloid leukemia (AML). Because CML cells express HLA-DR, they can be differentiated from normal bone marrow precursors. In embodiments of the invention involving an individual suspected of having CML, the cell population associated markers are selected from the group consisting of CD45 , CD34 to identify immature or "blast" cell populations, CD11b to identify normal and neoplastic myeloid cells, CD13, CD15 to identify normal and neoplastic myeloid cells, CD14, CD33, CD79a and b to identify normal and neoplastic B-cells, CD22, CD10, CD16, Bcr/Abl to identify malignant cells and terminal deoxyneucleotide transferase (TdT) to identify the differentiation state of normal and neoplastic cell populations.

As described herein, cell populations can be distinguished based on unique combinations of cell population associated markers, for example, cell surface markers, that are displayed on the cell surface. In the methods of the invention, the identification of normal and neoplastic internal cell populations based on the expression and level of cell population associated markers precedes the step of correlating the marker levels across each of the cell populations with the presence of at least one target protein. Thus, initially combinations of internal cell population associated markers are used to identify normal and neoplastic cell populations, which are subsequently correlated with the presence of at least one target protein. It is understood that the combinations of presence and expression level of cell population associated markers can be selected based on the particular cell populations internal to a biological sample that are desired to be identified for further correlation with the target protein of interest. It is further understood that, where the methods for quantification of staining based on comparison to internal cellular control populations can be generalized to the applications when reference populations are not present in the sample by exogenous addition, or spiking, of appropriate reference cellular populations into the sample.

The particular normal and neoplastic internal cell populations that are identified based on the expression and level of cell population associated markers for embodiments related to B-CLL, can encompass a population that is CD5 positive and CD19 positive; a population that is CD5 positive and CD20 negative, a population that is CD3 positive and CD56 positive; and a CD3 positive population. As an example of the principle of this invention, the normal or reactive T-cells (CD3+) and NK-cells (CD56+) are positive for the expression of ZAP-70 (protein), while normal B-cells (CD19+/CD5-) and normal granulocytes (identified by light scatter characteristics alone, or in conjunction with CD45) are negative for ZAP-70 (protein) expression. These cell populations internal to the sample are used to determine the level of expression of ZAP-70 protein in the B-CLL (neoplastic) population that is identified by being CD19+/CD5+ (potentially in conjunction with additional markers, e.g. FCM7-) relative to the internal positive and negative cell populations, and are used together to establish a composite profile.

In embodiments directed to CML, the cell population associated markers can identify a cell population that encompasses leukemic granulocytes and a further cell population that encompasses leukemic monocytes, in addition to any normal lymphocytes, monocytes or granulocytes. Thus, the present invention provides a method for establishing a composite marker profile for a sample derived from an individual suspected having CML by reacting the biological sample with a collection of binding molecules that contains two or more groups of binding molecules specific for distinct cell population associated markers, wherein marker levels and marker combinations identify normal and neoplastic cell populations internal to the sample; subsequently identifying the presence of normal and neoplastic cell populations internal to the sample by detecting the marker levels and marker combinations that identify the normal and neoplastic cell populations internal to the sample, and correlating the marker levels across each of the normal and neoplastic cell populations with the presence of at least one target protein to establish a composite marker profile for a sample derived from an individual suspected having Chronic Myelogenous Leukemia (CML). In this embodiment, the target protein can be selected from the group consisting of signal transduction proteins, proliferation markers, differentiation markers and apoptosis markers.

A biological sample can be processed for establishment of a composite marker profile using any cell preparative and staining procedures selected by the skilled person and appropriate for the particular sample. It is understood that the preparative and staining procedures, which generally include RBC lysis, staining, fixation, permeabilization, with some variation in the order of steps, can be used as long as the cell population associated markers of interest are maintained to allow identification of cell populations as described herein. Appropriate preparative procedures are well known in the art and described, for example, in red blood cell lysis of whole blood, bone marrow, ascites, etc. using hypotonic lysis (to obtain purified WBC populations), or the use of density-gradient techniques (e.g. Hypaque/Ficoll) to obtain mononuclear cell enriched specimens.

Thus, in a method of the invention for establishing a composite marker profile for a sample derived from an individual suspected having a neoplastic condition, the sample is initially reacted with collection of binding molecules that contains two or more groups of binding molecules specific for distinct cell population associated markers to identify normal and neoplastic cell populations internal to the sample. As set forth above, the groups of binding molecules can be selected by the user to identify normal and neoplastic cell populations internal to the sample. Once normal and neoplastic cell populations are identified, the cell populations are further correlated with the presence of a target protein to establish a composite marker profile.

As used herein, the term "target protein" refers to a protein that, as a result of its role in the mediation of cellular response, behavior or function across normal and neoplastic cell populations, can be used to establish a composite marker profile. In establishing a composite marker profile of the invention, one or more target proteins are generally measured in terms of their functional response to a modulatory stimulus, which is compared across normal and neoplastic cell populations internal to the sample. A target protein useful in a method of the invention can be, for example, a signaling molecule that mediates an effect related to metabolism, cell growth, differentiation, apoptosis, or fulfills any other physiological role. Accordingly, the modulation state of a target protein can be measured as it mounts a biological response as a specific or preferential substrate, molecular adapter, or component of a signaling pathway. In order to establish a composite marker profile of the present invention, the modulation state of the target protein, for example, its phosphorylation state, can be measured across neoplastic and normal cell populations internal to a sample.

A "composite marker profile" is used herein to refer to a matrix of expression levels of a target protein across compared across distinct cell populations internal to a biological sample. The composite marker profile represents a quantification of the target protein in a biological sample based on standardization of the target protein expression in neoplastic cells against the target protein staining levels in normal cell populations positive for the target protein. The normal cell populations positive for the target protein serve as positive controls of varying target protein expression levels. One or more internal normal cell population that do not express the target protein can serve as a negative control. In tissue samples, peripheral blood contamination can cause sufficient granulocytes to be present to serve as an internal negative control.

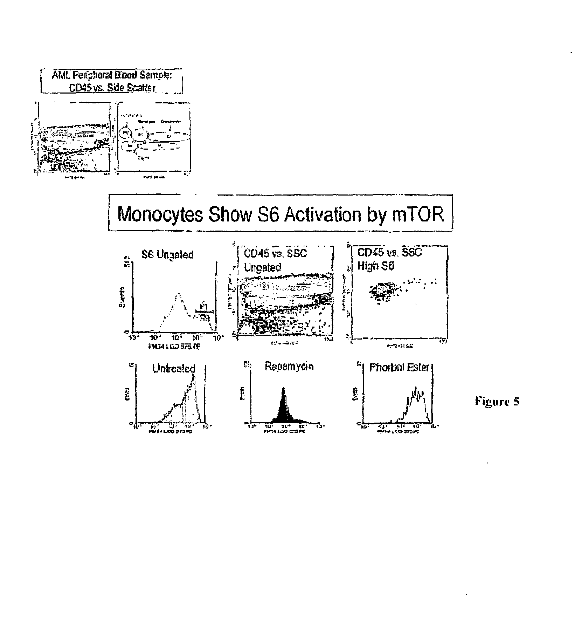

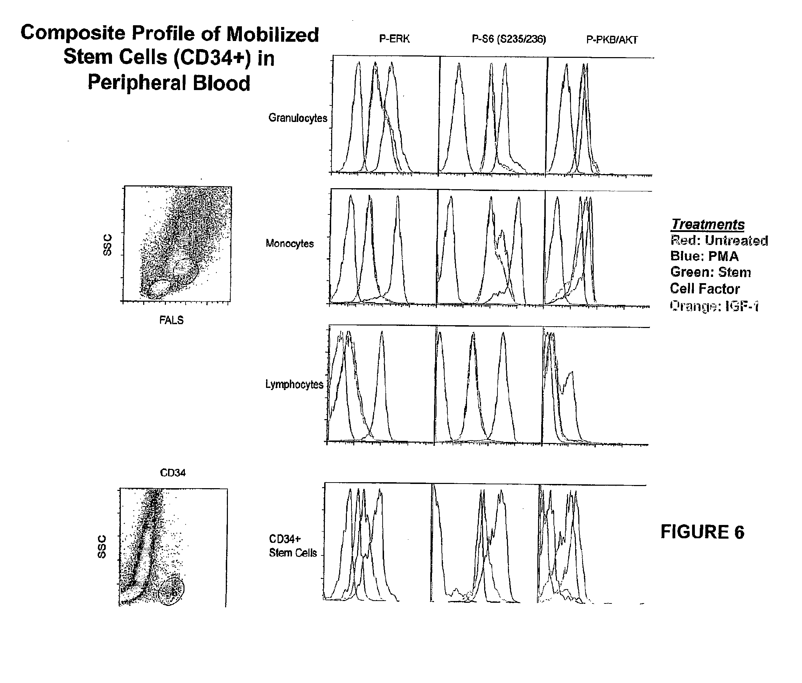

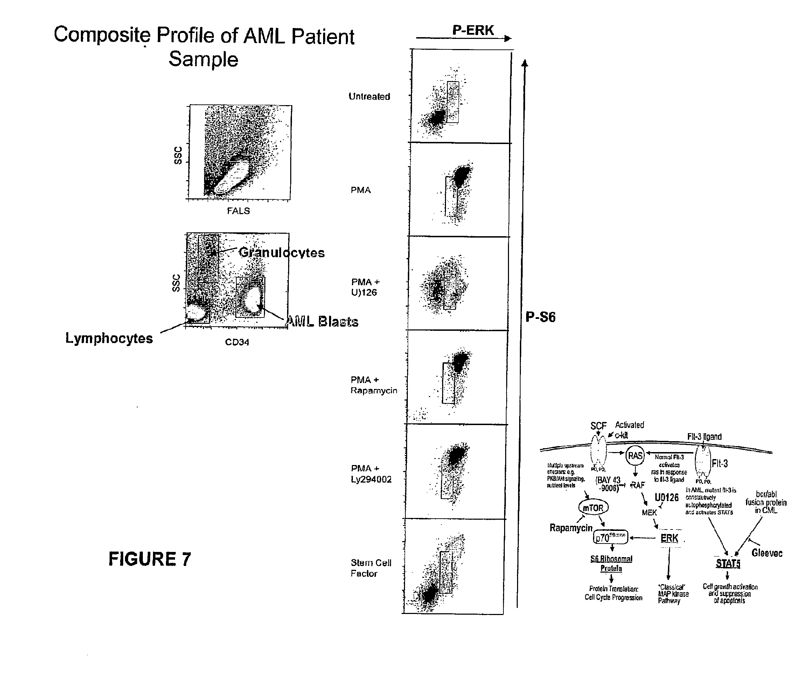

In a method for establishing a composite marker profile for a sample derived from an individual suspected having AML or CML, the target protein can be a signal transduction protein, for example, Abl, CRKL, Hck, STAT1, STAT3, STAT5, Flt3, Akt/PKB, ERK, mTOR or S6. Figure 5 shows flow cytometry panels depicting S6 activation by mTOR in a AML sample. Additionally, in a method for establishing a composite marker profile for a sample derived from an individual suspected having CML, the target protein can be a proliferation marker, for example, Cyclin D1 or Cyclin A2, as well as an apoptosis marker, for example, cleaved-Caspase-3 or Bcl-X1. Figure 6 shows a composite profile of mobilized stem cells in peripheral blood established by comparing the responses of three different signal transduction proteins, P-ERK, P-S6, and P-PKB/AKT in CD34+ stem cells to those of the internal reference populations (lymphocytes, granulocytes and monocytes) to three different stimuli. Figure 7 depicts a composite profile established according to the invention of an AML patient sample by measuring the expression of the signal transduction target proteins P-ERK and P-S6 expression in the blood sample and comparing the expression in AML blasts (red), with that observed in internal reference populations of lymphocytes (blue) and granulocytes (green).

In a particular embodiment of the invention, quantification of ZAP-70 in B-CLL can be accomplished by standardizing against the ZAP-70 staining levels in normal T and natural killer cells, both of which are positive for ZAP-70. In this embodiment, which is directed to establishing a composite marker profile for a sample derived from an individual suspected having B-CLL, the normal T and natural killer cells provide two positive controls of varying intensity with the natural killer cells having brighter staining than the T cells. In this embodiment, granulocytes and normal B-cells in the peripheral blood and bone marrow biological samples are ZAP-70 negative and can serve as negative controls.

In particular embodiments of the methods disclosed herein, the target protein is detected for modifications, for example, phosphorylation state, that can be correlated to a condition. Cells rely, to a great extent, on extracellular molecules as a means by which to receive stimuli from their immediate environment. These extracellular signals are essential for the correct regulation of such diverse cellular processes as differentiation, contractility, secretion, cell division, contact inhibition, and metabolism and can be correlated to abnormal or potentially deleterious processes such as virus-receptor interaction, inflammation, and cellular transformation to a cancerous state. A central feature of this process, referred to as signal transduction, is the reversible phosphorylation of certain proteins. Phosphorylation is a dynamic process involving competing phosphorylation and dephosphorylation reactions, and the level of phosphorylation at any given instant reflects the relative activities, at that instant, of the protein kinases and phosphatases that catalyze these reactions.

Therefore, a composite marker profile of the invention can correlate the phosphorylation or dephosphorylation of a target protein, rather than merely its expression, since such conformational changes in regulated proteins alter their biological properties. While the majority of protein phosphorylation occurs at serine and threonine amino acid residues, phosphorylation at tyrosine residues also occurs, and has begun to attract a great deal of interest since the discovery that many oncogene products and growth factor receptors possess intrinsic protein tyrosine kinase activity. The importance of protein tyrosine phosphorylation in growth factor signal transduction, cell cycle progression and neoplastic transformation is now well established (Cantley et al., 1991, Cell 64:281-302; Hunter T., 1991, Cell 64:249-270; Nurse, 1990, Nature 344:503-508; Schlessinger et al., 1992, Neuron 9:383-391; Ullrich et al., 1990, Cell 61:203-212). Subversion of normal growth control pathways leading to oncogenesis has been shown to be caused by activation or overexpression of protein tyrosine kinases which constitute a large group of dominant oncogenic proteins (reviewed in Hunter, T., 1991, Cell 64:249-270). As disclosed herein, a composite marker profile of the invention can correlate the phosporylation state of a target protein across each of the normal and neoplastic cell populations with the presence of a target protein to establish a composite marker profile for a sample derived from an individual suspected having a neoplastic condition.

In a preferred embodiment, all of the cell populations that make up the composite marker profile are internal to the biological sample. As used herein, the term "internal" when used in reference to a biological sample means that all of the cell populations are initially present in the sample, rather than added or "spiked" into the sample prior to establishment of the composite marker profile. In the absence of a cell population that can serve as a negative control, the comparative matrix established by comparison across the internal populations of normal cells and neoplastic cells positive for the target protein can semi-quantitative determination of target protein levels sufficient to establish a composite marker profile according to the methods disclosed herein. It is further contemplated that internal cellular control populations, positive and negative, can be added to a biological sample in cases where such control populations are not internal to the sample.

As described above, the invention provides a method for establishing a composite marker profile for a sample derived from an individual suspected having B-Cell Chronic Lymphocytic Leukemia (B-CLL) by reacting the biological sample with a collection of binding molecules containing two or more groups of binding molecules specific for distinct cell population associated markers and identifying the presence of normal and neoplastic cell populations internal to the sample by detecting the marker levels and marker combinations that identify the normal and neoplastic cell populations internal to the sample, and correlating the marker levels across each of the normal and neoplastic cell populations with the presence of ZAP-70 to establish a composite marker profile for a sample derived from an individual suspected having B-Cell Chronic Lymphocytic Leukemia (B-CLL).

Quantification of ZAP-70 in B-CLL can be accomplished by standardized against the ZAP-70 staining levels in normal T and natural killer cells, both of which are positive for ZAP-70. This provides two positive controls of varying intensity with the natural killer cells having brighter staining than the T cells. In this embodiment, granulocytes or normal B-cells in the peripheral blood and bone marrow biological samples are ZAP-70 negative and can serve as negative controls.

In tissue samples, peripheral blood contamination can cause sufficient granulocytes to be present to serve as an internal negative control. When not present, the ratio reference to the normal T cells is adequate for "semi-quantitative" determination. A specific combination of antigens in addition to ZAP-70 to accomplish this in CLL is CD 19, CD5, and CD56. Other antibody combinations could be utilized for selection of relevant, both malignant and normal, populations. In addition to those mentioned above, this might include, but are not limited to, CD3, CD20, CD23, CD79a, MCF7 and the immunoglobulin light chains. The methods disclosed herein can be practiced with samples specifically stained for a given target protein, surface or intracellular, where normal cell populations both positive and negative for the same target protein, or stainable cell property, are present and can serve as a biological reference for determining if a cell population of interest is positive or not for the same antigen or marker. Internal biological cell population also serve by comparison to determine the semi-quantitative level of this antigen or marker in the cells of interest. The internal cell populations can be selected based on other cellular properties that include but are not restricted to antigen detection and scattered light properties. Specific markers needed will be dependent on the specific application of the invention method and selected by the skilled person accordingly.

According to the embodiments described above for methods of preparing composite marker profiles for particular hematologic cancers can be extended to diagnostic methods. A composite marker profile according to the methods of the invention can be used to diagnose a neoplastic condition in an individual suspected to be afflicted of a neoplastic condition by comparing the composite marker profile to one or more reference composite marker profiles, wherein the comparison allows for predicting the clinical course of the neoplastic condition.

As used herein, the phrase "predicting the clinical course" is meant to encompass diagnosis as well as prognosis of any factor related to the condition or treatment, including, survivorship, prognosis of disease course and prognosis of response to treatment.

As used herein, the term "reference" when used in the context of a composite marker profile refers to a composite marker profile known to be associated with a particular parameter against which the composite marker profile obtained from the biological sample being tested is compared. A reference composite marker profile can represent a particular neoplastic condition, a stage of a neoplastic condition or can be representative of a known disease course, for example, aggressive versus indolent course. For example, a reference composite marker for a diagnostic method of the invention can represent the particular condition with which the individual is suspected to be afflicted. A reference composite marker profile can represent a particular stage of the neoplastic condition or can be representative of a known disease course, for example, aggressive course.

In preferred embodiments of the diagnostic and prognostic methods of the invention, an individual's composite marker profile is compared to more than one reference composite marker profile. For example, in a diagnostic application an individual's composite marker profile can be compared to a collection of two or more reference composite marker profile that represent a spectrum of clinical stages so as to enable a nuanced classification of the individual's disease stage by comparison. Similarly, in a prognostic application an individual's composite marker profile can compared to a collection of two or more reference composite marker profile that represent a spectrum from non-aggressive to aggressive or a spectrum from treatment responsive to non-responsive.

A composite marker profile according to the methods of the invention can be used to diagnose a hematologic cancer in an individual suspected to be afflicted of a hematologic cancer by comparing a composite marker profile of the obtained by the methods described above to one or more reference composite marker profiles, wherein the comparison allows for predicting the clinical course of the hematologic cancer, which is selected from the group consisting of Chronic Lymphocytic Leukemia (CLL), Acute Myelogenous Leukemia (AML), Chronic Myelogenous Leukemia (CML), and Acute Lymphocytic Leukemia (ALL).

In one embodiment, a composite marker profile according to the methods of the invention can be used to diagnose Ig-unmutated B-cell Chronic Lymphocytic Leukemia (CLL) in an individual suspected to be afflicted by comparing the composite profile to one or more reference composite profiles, wherein the comparison allows for predicting the clinical course of Ig-unmutated B-cell Chronic Lymphocytic Leukemia (CLL) (Hamblin et al., Blood 94:1848-1854 (1999)). The invention provides a method for predicting the clinical course of Ig-unmutated B-cell Chronic Lymphocytic Leukemia (CLL) in an individual by providing a biological sample derived from the individual; reacting the biological sample with a collection of binding molecules that contains two or more groups of binding molecules specific for distinct cell population associated markers, wherein marker levels and marker combinations identify normal and neoplastic cell populations internal to the sample; identifying the presence of normal and neoplastic cell populations internal to the sample by detecting the marker levels and marker combinations that identify the normal and neoplastic cell populations internal to the sample; correlating the marker levels across each of the normal and neoplastic cell populations with the presence of ZAP-70 to establish a composite marker profile for a sample derived from an individual suspected having B-Cell Chronic Lymphocytic Leukemia (B-CLL), wherein the composite profile represents a relative quantification of ZAP-70 levels; and comparing the composite profile to one or more reference composite profiles, wherein the comparison allows for predicting the clinical course of Ig-unmutated B-cell Chronic Lymphocytic Leukemia (CLL).

In further embodiments, the invention provides a method for predicting the clinical course of Ig-unmutated B-cell Chronic Lymphocytic Leukemia (CLL) in an individual by providing a biological sample derived from the individual; reacting the biological sample with a collection of binding molecules that contains two or more groups of binding molecules specific for distinct cell population associated markers, wherein marker levels and marker combinations identify normal and neoplastic cell populations internal to the sample; identifying the presence of normal and neoplastic cell populations internal to the sample by detecting the marker levels and marker combinations that identify the normal and neoplastic cell populations internal to the sample; correlating the marker levels across each of the normal and neoplastic cell populations with the presence of a target protein selected from the group consisting of ZAP-70, Activation Induced C-type Lectin (AICL) and Lipoprotein Lipase to establish a composite marker profile for a sample derived from an individual suspected having B-Cell Chronic Lymphocytic Leukemia (B-CLL), wherein the composite profile represents a relative quantification of the target protein level; and comparing the composite profile to one or more reference composite profiles, wherein the comparison allows for predicting the clinical course of Ig-unmutated B-cell Chronic Lymphocytic Leukemia (CLL).

In one embodiment, a composite marker profile according to the methods of the invention can be used to diagnose Ig-mutated B-cell Chronic Lymphocytic Leukemia (CLL) in an individual suspected to be afflicted by comparing the composite profile to one or more reference composite profiles, wherein the comparison allows for predicting the clinical course of Ig-mutated B-cell Chronic Lymphocytic Leukemia (CLL). In further embodiments, the invention provides a method for predicting the clinical course of Ig-mutated B-cell Chronic Lymphocytic Leukemia (CLL) in an individual by providing a biological sample derived from the individual; reacting the biological sample with a collection of binding molecules that contains two or more groups of binding molecules specific for distinct cell population associated markers, wherein marker levels and marker combinations identify normal and neoplastic cell populations internal to the sample; identifying the presence of normal and neoplastic cell populations internal to the sample by detecting the marker levels and marker combinations that identify the normal and neoplastic cell populations internal to the sample; correlating the marker levels across each of the normal and neoplastic cell populations with the presence of a target protein selected from the group consisting of IM68532, IM 1286077, and LC15506 to establish a composite marker profile for a sample derived from an individual suspected having B-Cell Chronic Lymphocytic Leukemia (B-CLL), wherein the composite profile represents a relative quantification of the target protein level; and comparing the composite profile to one or more reference composite profiles, wherein the comparison allows for predicting the clinical course of Ig-mutated B-cell Chronic Lymphocytic Leukemia (CLL).

In a further embodiment, a composite marker profile according to the methods of the invention can be used to diagnose CML in an individual suspected to be afflicted with CML by comparing a composite marker profile obtained by the methods disclosed above to one or more reference composite profiles, wherein the comparison allows for predicting the clinical course of CML. In an additional embodiment, a composite marker profile obtained by the methods disclosed above can be used to diagnose AML in an individual suspected to be afflicted with AML by comparing a composite profile to one or more reference composite profiles, wherein the comparison allows for predicting the clinical course of AML. In yet another diagnostic embodiment, a composite marker profile obtained by the methods disclosed above can be used to diagnose ALL in an individual suspected to be afflicted with ALL by comparing a composite profile to one or more reference composite profiles, wherein the comparison allows for predicting the clinical course of ALL.

In a further embodiment of the invention, the methods of preparing composite marker profiles for particular hematologic cancers can be extended to prognostic methods. In particular, a composite profile obtained by the methods disclosed above can be used to select the appropriate clinical management and treatment modalities for an individual afflicted with a neoplastic condition.

A composite marker profile according to the methods of the invention can be useful to identify individuals who are predicted to effectively respond to a particular therapy from individuals who would have never progressed to a more advanced stage of the disease regardless of treatment. Accordingly, a composite profile of the invention can be used to predict an individual's clinical course, notwithstanding the conventional stage of the disease, and aid clinicians in better evaluating treatment options, as well as greatly enhance the value of clinical studies by better distinguishing the effects of a particular treatment.

A composite marker profile according to the methods of the invention can be used to predict the clinical progression of any neoplastic condition in an individual suspected to be afflicted of a neoplastic condition by comparing the composite marker profile to one or more reference composite marker profiles, wherein the comparison allows for predicting the clinical progression of the neoplastic condition. A composite marker profile according to the methods of the invention can be used to predict the clinical progression of a hematologic cancer in an individual suspected to be afflicted of a hematologic cancer by comparing a composite marker profile obtained by the methods described above to one or more reference composite marker profiles, wherein the comparison allows for predicting the clinical course of the hematologic cancer, which is selected from the group consisting of Chronic Lymphocytic Leukemia (CLL), Acute Myelogenous Leukemia (AML), Chronic Myelogenous Leukemia (CML), and Acute Lymphocytic Leukemia (ALL).

In one embodiment, a composite marker profile according to the methods of the invention can be used to predict the clinical progression of Ig-unmutated B-cell Chronic Lymphocytic Leukemia (CLL) in an afflicted individual by comparing the composite profile to one or more reference composite profiles, wherein the comparison allows for predicting the clinical progression of Ig-unmutated B-cell Chronic Lymphocytic Leukemia (CLL). The invention thus provides a method for predicting the clinical progression of Ig-unmutated B-cell Chronic Lymphocytic Leukemia (CLL) in an individual by providing a biological sample derived from the individual; reacting the biological sample with a collection of binding molecules that contains two or more groups of binding molecules specific for distinct cell population associated markers, wherein marker levels and marker combinations identify normal and neoplastic cell populations internal to the sample; identifying the presence of normal and neoplastic cell populations internal to the sample by detecting the marker levels and marker combinations that identify the normal and neoplastic cell populations internal to the sample; correlating the marker levels across each of the normal and neoplastic cell populations with the presence of ZAP-70 to establish a composite marker profile for a sample derived from the individual afflicted with B-Cell Chronic Lymphocytic Leukemia (B-CLL), wherein the composite profile represents a relative quantification of ZAP-70 levels; and comparing the composite profile to one or more reference composite profiles, wherein the comparison allows for prediction of the clinical progression of Ig-unmutated B-cell Chronic Lymphocytic Leukemia (CLL).

A composite marker profile according to the methods of the invention can be useful to allow the user to choose an aggressive or experimental treatment for an individual by allowing determination of whether such treatment will result in tangible benefit or is worth the associated risks. For example, some B-CLL patients succumb to the combined effects of treatment and B-CLL rather than to the effects of B-CLL alone. A composite marker profile according to the methods of the invention can be used to select more aggressive treatment, such as radiation therapy, chemotherapy, transplants and immunotherapy, by allowing to identify B-CLL patients already in the advanced stages of B-CLL without relying on conventional Rai and Binet staging systems.

In a further embodiment, a composite marker profile according to the methods of the invention can be used to predict the clinical progression of CML in an individual afflicted with CML by comparing a composite marker profile obtained by the methods disclosed above to one or more reference composite profiles, wherein the comparison allows for prediction of the clinical progression of CML. In an additional embodiment, a composite marker profile obtained by the methods disclosed above can be used to predict the clinical progression of AML in an individual afflicted with AML by comparing a composite profile to one or more reference composite profiles, wherein the comparison allows for prediction of the clinical progression AML. In yet another diagnostic embodiment, a composite marker profile obtained by the methods disclosed above can be used to predict the clinical progression of CLL in an individual suspected to be afflicted with CLL by comparing a composite profile to one or more reference composite profiles, wherein the comparison allows for prediction of the clinical progression of CLL.

In yet another embodiment, the methods of the invention allow for establishment of a composite profile that is useful on monitoring the ongoing treatment of an individual afflicted with a hematological cancer. For example, in a CML patient, a composite profile can be useful to monitor the response to treatment with Gleevec, while in an AML patient response to treatment with rapamycin, which inhibits mTOR can be monitored. Furthermore, a composite profile can be useful in drug development as well as the development of molecularly targeted therapies. It is understood by those of skill in the art apprized of the instant disclosure that there are many further useful applications of the method of the invention for establishing a composite profile, including, for example, in determining the relative importance of different signal transduction pathways in the development and/or progression of disease in individual patients so as to allow for personalized therapy.

The following examples are intended to illustrate but not limit the present invention.

### EXAMPLE I

### Preparation of Samples for Establishing Composite Profile

This Example shows preparation of a sample for establishing a composite profile for an individual suspected of having having B-Cell Chronic Lymphocytic Leukemia (B-CLL).

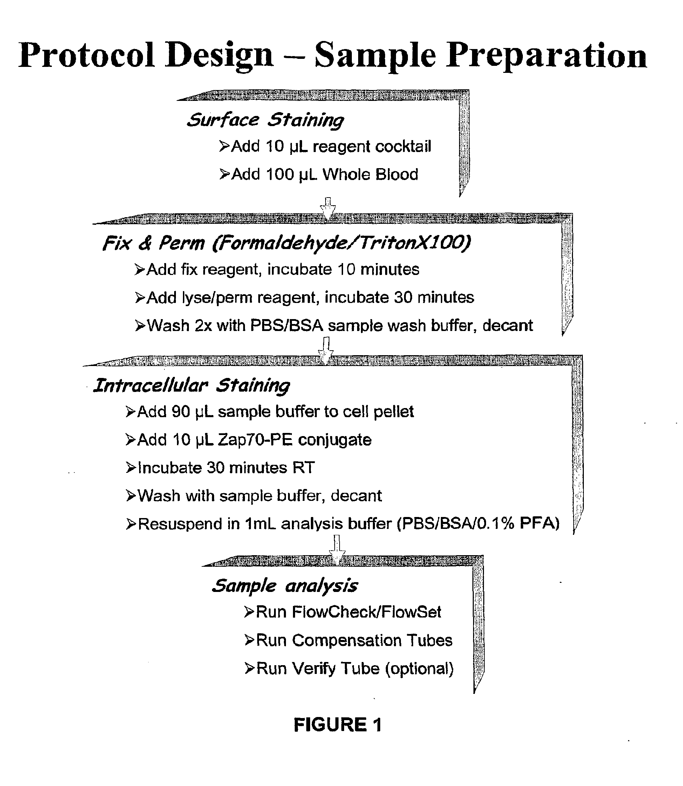

Briefly, 10 µl of a reagent mix containing the cell surface markers CD5-FTTC, plus CD3-ECD, plus CD56-PC5, plus CD19-PC7 was placed in a tube and 100 µl of whole blood were added. After a 20 minute incubation at room temperature, 64 µl of fixative (10% formaldehyde) were added followed by incubation at room temp for 10 minutes. Next, 1 ml of lyse/perm reagent (0.1% Triton X-100) was added, followed by incubation for 30 minutes at room temperature and subsequent centrifugation. The cells were then washed twice with buffer (PBS with 2% BSA), resuspended in 10µl anti-ZAP-70-PE plus 90 µl buffer, incubated at room temp for 30 min, then washed once with buffer, and resuspended in buffer containing 0.1% paraformaldehyde. The sample is then analysed on a flow cytometer. Figure 1 shows a schematic diagram that exemplifies sample preparation for methods for establishing a composite marker profile.

### EXAMPLE II

### Analysis of Sample for Establishing Composite Profile

This example shows analysis of the sample by flow cytometry to establish a composite profile.

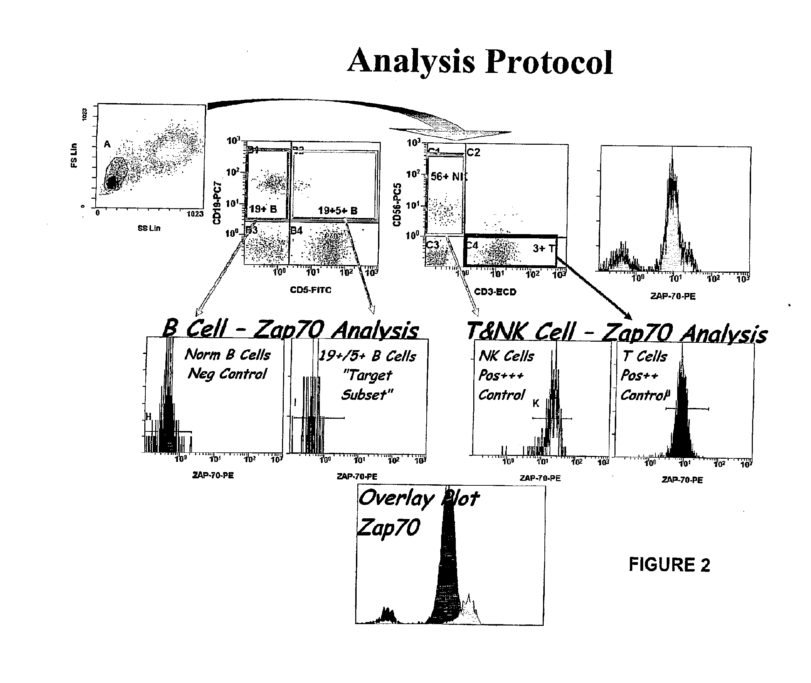

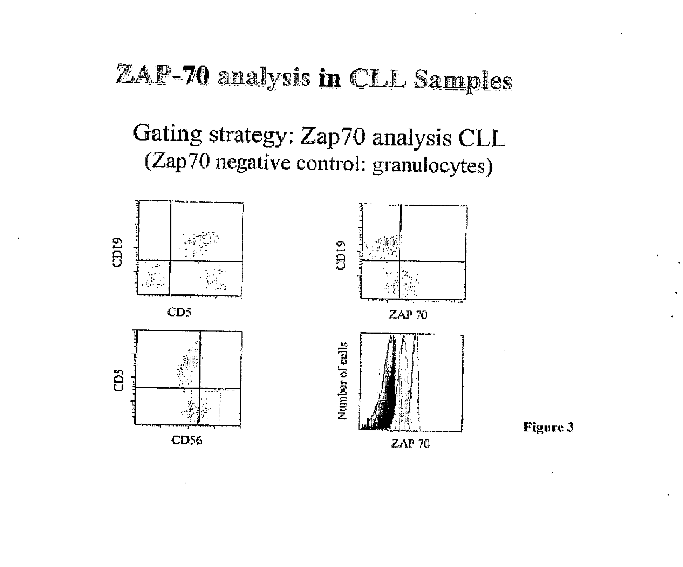

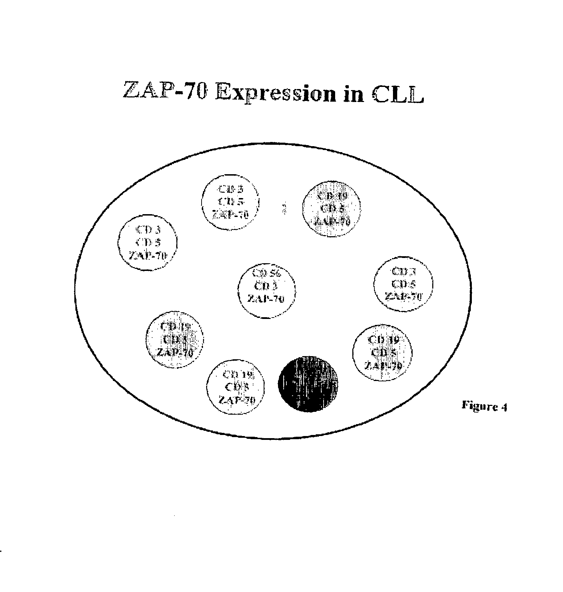

As shown in Figure 2, a composite profile was established using a ZAP-70 gating/analysis algorithm as follows: The first gate of a normal whole blood sample identifies lymphocytes (blue plus green) by light scatter in the upper left histogram. If desired, CD45 versus side scatter (linear scale) can be used to set gates around CD45-bright lymphocytes in peripheral blood for CD4 enumeration, and CD45 versus side scatter (log scale) can be used to identify blast populations in bone marrow for phenotyping. In Figure 2, the CD19 versus CD5 histogram identifies normal B-cells (purple, upper left quadrant), normal T-cells (blue, lower right) and normal peripheral blood "pre" B-cells (CD19+5 +, upper right quadrant). The B-CLL neoplastic cells also fall in the upper right quadrant, but contrary to their normal counterpart, the B-CLL neoplastic cells do not express ZAP-70. The Overlay plot shown at the bottom of Figure 2, summarizes the composition profile and shows where the B-CLL cells fall in this histogram (ZAP-70 expression) in relation to internal normal B-cells (CD 19+), normal T-cells (CD5+3+) and NK cells (CD56+). Figure 3 shows flow cytometry panels obtained from a gating strategy for ZAP-70 analysis in CLL sample. Figure 4 shows a diagram depicting ZAP-70 expression in the universe of cells present in a CLL sample. (red: B-cells; green: normal T-cells; purple: NK cells)

Figure 5 shows flow cytometry panels depicting S6 activation by mTOR in a AML sample.

Figure 6 shows a Composite Profile of Mobilized Stem Cells (CD34+) in Peripheral Blood. The histograms provide a comparison of the responses of of three different signal transduction proteins, P-ERK, P-S6, and P-PKB/AKT in each of mobilized stem cells, normal granulocytes, normal monocytes and normal lymphocytes after stimulation with PMA (blue), Stem Cell Factor (green), or IGF-1 (orange), compared to untreated sample (red). shows a (human) whole blood sample first treated in *vivo* to mobilize stem cells (CD34+). The two histograms on the left show how the CD34+ stem cells (bottom) and normal Granulocytes, Monocytes and Lymphocytes (top) are identified. For these experiments, multiple samples from this single donor are treated (in vitro) with factors which stimulate signal transduction pathways: PMA (blue), Stem Cell Factor (green), or IGF-1 (orange), compared to untreated sample (red). The sets of histograms on the right side show the response (or lack) of three different signal transduction proteins, P-ERK, P-S6, and P-PKB/AKT. The results compare the responses of the CD34+ stem cells to those of the internal reference populations (lymphocytes, granulocytes and monocytes), and demonsrate the CD34+ cell response to these stimuli.

Figure 7 shows a composite profile of a single AML Patient. Blood aliquots are stimulated with PMA, with or without specific signal transduction pathway inhibitors ("map" in lower right corner), or with Stem Cell Factor. The responses are measured as bivariate plots of P-ERK versus P-S6 (right panels), where the red population is the AML blasts, the blue the internal lymphocytes, and the green, the internal granulocytes. The AML blasts (red), Lymphocytes (blue) and Granulocytes (green) are identified using CD34 versus SSC as depicted in the two boxes on the left. Levels of P-ERK and P-S6 expression are shown in untreated (starting from top), PMA treated, PMA + U0126 (P-ERK Inhibitor), PMA + Rapamycin (mTOR> S6 inhibitor), PMA + Ly294002 (PKB/AKT Inhibitor) and Stem Cell Factor treated (bottom) whole blood samples. In all P-epitope histograms, box indicates level of P-ERK and P-S6 in unstimulated sample, indicating "baseline" expression. Histograms show the effect of different treatments on AML blast cells (red) compared to lymphocytes (blue) and granulocytes (green).

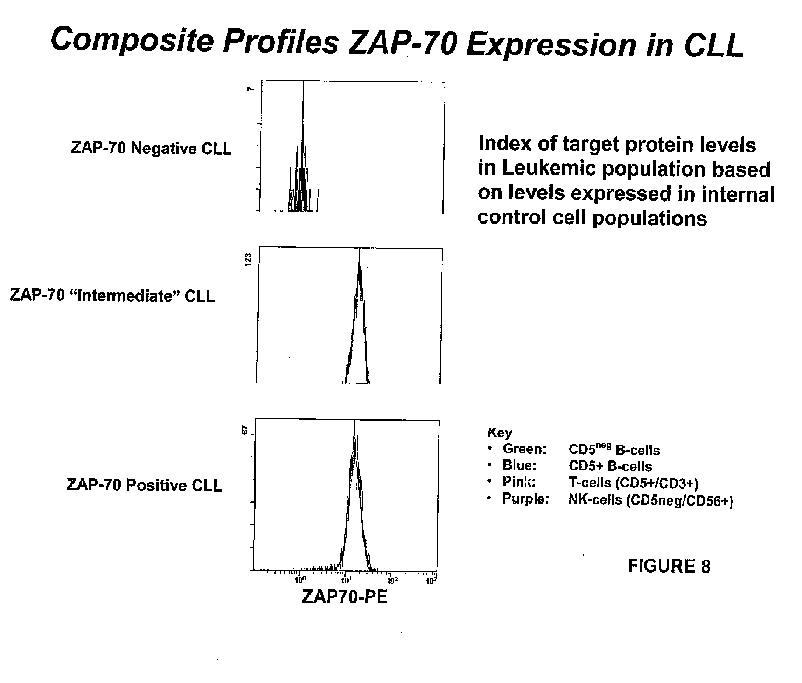

Figure 8 shows a CLL composite profile. While, the B-cell negative peak and T-cell positive peaks are consistent between specimen, NK-cell positive peak expression is higher in ZAP70 high positive specimen, with less consistency between specimens. ZAP70⁺ CLL had 0.2% "normal B-cells", but the rare events clustered equivalent to the negative peaks in other specimen. A clear shift is seen in CLL with intermediate ZAP70 from normal B-cells -S/N 2.15

## Claims

1. A method for establishing a composite marker profile for a sample derived from an individual suspected having a neoplastic condition, the method comprising the steps of:
(a) reacting the biological sample with a collection of binding molecules, wherein the collection of binding molecules contains two or more groups of binding molecules specific for distinct cell population associated markers, wherein marker levels and marker combinations identify normal and neoplastic cell populations internal to the sample,
(b) identifying the presence of normal and neoplastic cell populations internal to the sample by detecting the marker levels and marker combinations that identify the normal and neoplastic cell populations internal to the sample, and
(c) correlating the marker levels across each of the normal and neoplastic cell populations with the presence of a target protein to establish a composite marker profile for a sample derived from an individual suspected having a neoplastic condition, wherein the target protein is a signaling molecule.

2. The method of claim 1, wherein said target protein is an activation-phosphorylated signal transduction protein.

3. The method of claim 1, further comprising a determination of target protein modification.

4. The method of claim 1, wherein the neoplastic condition is selected from the group consisting of Chronic Lymphocytic Leukemia (CLL), Acute Myelogenous Leukemia (AML), Chronic Myelogenous Leukemia (CML), and Acute Lymphocytic Leukemia (ALL).

5. The method of claim 1, wherein the cell population associated markers comprise cell-surface markers selected from the group consisting of CD3, CD5, CD10, CD11b, CD13, CD 15, CD 14, CD 15, CD16, CD 19, CD22, CD 23, CD56, CD45, CD33, CD34, CD15, CD16, MPL (myeloperoxidase), CD64, CD79a, CD79b, and CD117 (c-kit receptor).

6. The method of claim 5, wherein the markers further include kappa and lambda immunoglobulin light chains to determine clonality.

7. The method of claim 4, wherein said cancer is B-Cell Chronic Lymphocytic Leukemia (B-CLL).

8. The method of claim 7, wherein said target protein is selected from the group consisting of ZAP-70, Activation Induced C-type Lectin (AICL), Lipoprotein Lipase and IM68532.

9. The method of claim 8, wherein the B-Cell Chronic Lymphocytic Leukemia (B-CLL) is Ig-mutated B-CLL.

10. The method of claim 7, wherein the cell population associated markers are selected from the group consisting of CD3, CD5, CD19, CD 23, CD38, CD56, CD 79b, and fmc 7.

11. The method of claim 10, wherein the cell population associated markers identify a cell population comprising leukemic B cells.

12. The method of claim 11, wherein the leukemic B cells are ZAP-70 positive.

13. The method of claim 10, wherein one or more of the cell populations comprise ZAP-70 negative cell populations.

14. The method of claim 10, wherein one or more of the cell populations comprise normal B cells.

15. The method of claim 13, wherein the ZAP-70 negative cell population comprises granulocytes.

16. A method for predicting the clinical course of Ig-unmutated B-cell Chronic Lymphocytic Leukemia (CLL) in an individual, the method comprising the steps of :
(a) providing a biological sample derived from the individual;
(b) reacting the biological sample with a collection of binding molecules, wherein the collection of binding molecules contains two or more groups of binding molecules specific for distinct cell population associated markers, wherein marker levels and marker combinations identify normal and neoplastic cell populations internal to the sample,
(c) identifying the presence of normal and neoplastic cell populations internal to the sample by detecting the marker levels and marker combinations that identify the normal and neoplastic cell populations internal to the sample,
(d) correlating the marker levels across each of the normal and neoplastic cell populations with the presence of ZAP-70 to establish a composite marker profile for a sample derived from an individual suspected having B-Cell Chronic Lymphocytic Leukemia (B-CLL), wherein the composite profile represents a relative quantification of ZAP-70 levels; and
(e) comparing the composite profile to one or more reference composite profiles, wherein the comparison allows for predicting the clinical course of Ig-unmutated B-cell Chronic Lymphocytic Leukemia (CLL).

17. A method for establishing a composite marker profile for a sample derived from an individual suspected having Chronic Myelogenous Leukemia (CML), the method comprising the steps of:
(a) reacting the biological sample with a collection of binding molecules, wherein the collection of binding molecules contains two or more groups of binding molecules specific for distinct cell population associated markers, wherein marker levels and marker combinations identify normal and neoplastic cell populations internal to the sample,
(b) identifying the presence of normal and neoplastic cell populations internal to the sample by detecting the marker levels and marker combinations that identify the normal and neoplastic cell populations internal to the sample, and
(c) correlating the marker levels across each of the normal and neoplastic cell populations with the presence of at least one target protein selected from the group consisting of activation-phosphorylated signal transduction proteins, proliferation markers, differentiation markers and apoptosis markers to establish a composite marker profile for a sample derived from an individual suspected having Chronic Myelogenous Leukemia (CML).

18. The method of claim 17, wherein the cell population associated markers are selected from the group consisting of CD45, CD34, CD11b, CD13, CD15, CD14, CD33, CD79a, CD79b, CD22, CD10, CD16, Bcr/Abl and TdT.

19. The method of claim 17, the target protein is an activation-phosphorylated signal transduction protein.

20. The method of claim 19, the activation-phosphorylated signal transduction protein is selected from the group consisting of Abl, CRKL, Hck, STAT1, STAT3, STAT5, Akt/PKB, and S6.

21. The method of claim 17, the target protein is a proliferation marker or an apoptosis marker

22. The method of claim 21, wherein the target protein is a proliferation marker is selected from the group consisting of Cyclin D1 and Cyclin A2.

23. The method of claim 21, wherein the target protein is an apoptosis marker selected from the group consisting of Caspase-3 and Bcl-X1.

24. A method for predicting the clinical course of Chronic Myelogenous Leukemia (CML)in an individual, the method comprising the steps of:
(a) providing a biological sample derived from the individual;
(b) reacting the biological sample with a collection of binding molecules, wherein the collection of binding molecules contains two or more groups of binding molecules specific for distinct cell population associated markers, wherein marker levels and marker combinations identify normal and neoplastic cell populations internal to the sample,
(c) identifying the presence of normal and neoplastic cell populations internal to the sample by detecting the marker levels and marker combinations that identify the normal and neoplastic cell populations internal to the sample,
(d) correlating the marker levels across each of the normal and neoplastic cell populations with the presence of at least one target protein selected from the group consisting of activation-phosphorylated signal transduction proteins, proliferation markers and apoptosis markers to establish a composite marker profile for a sample derived from an individual suspected having Chronic Myelogenous Leukemia (CML),and
(e) comparing the composite profile to one or more reference composite profiles, wherein the comparison allows for predicting the clinical course of Chronic Myelogenous Leukemia (CML).

25. A method for establishing a composite marker profile for a sample derived from an individual suspected having Acute Myelogenous Leukemia (AML), the method comprising the steps of:
(a) reacting the biological sample with a collection of binding molecules, wherein the collection of binding molecules contains two or more groups of binding molecules specific for distinct cell population associated markers, wherein marker levels and marker combinations identify normal and neoplastic cell populations internal to the sample,
(b) identifying the presence of normal and neoplastic cell populations internal to the sample by detecting the marker levels and marker combinations that identify the normal and neoplastic cell populations internal to the sample, and
(c) correlating the marker levels across each of the normal and neoplastic cell populations with the presence of at least one target protein selected from the group consisting of activation-phosphorylated signal transduction proteins, proliferation markers and apoptosis markers to establish a composite marker profile for a sample derived from an individual suspected having Acute Myelogenous Leukemia (AML).

26. The method of claim 25, wherein the cell population associated markers are selected from the group consisting of CD45, CD33, CD34, CD11b, CD13, CD14, CD15, CD16, MPL (myeloperoxidase), CD64 and CD117 (c-kit receptor).

27. The method of claim 26, wherein the cell population associated markers identify a cell population comprising leukemic granulocytes or leukemic monocytes.

28. The method of claim 25, wherein the target protein is an activation-phosphorylated signal transduction protein.

29. The method of claim 25, wherein the activation-phosphorylated signal transduction protein is selected from the group consisting of Abl, CRKL, Hck, STAT1, STAT3, STAT5, Akt/PKB, and S6.

30. The method of claim 25, wherein the target protein is a proliferation marker or an apoptosis marker.

31. The method of claim 30, wherein the target protein is a proliferation marker selected from the group consisting of Cyclin D1 and Cyclin A2.

32. The method of claim 30, wherein the target protein is an apoptosis marker selected from the group consisting of Caspase-3 and Bcl-Xl.

33. A method for predicting the clinical course of Acute Myelogenous Leukemia (AML) in an individual, the method comprising the steps of :
(b) reacting the biological sample obtained from the individual with a collection of binding molecules, wherein the collection of binding molecules contains two or more groups of binding molecules specific for distinct cell population associated markers, wherein marker levels and marker combinations identify normal and neoplastic cell populations internal to the sample,
(c) identifying the presence of normal and neoplastic cell populations internal to the sample by detecting the marker levels and marker combinations that identify the normal and neoplastic cell populations internal to the sample,
(d) correlating the marker levels across each of the normal and neoplastic cell populations with the presence of at least one target protein selected from the group consisting of activation-phosphorylated signal transduction proteins, proliferation markers and apoptosis markers to establish a composite marker profile for a sample derived from an individual suspected having Acute Myelogenous Leukemia (AML); and
(e) comparing the composite profile to one or more reference composite profiles, wherein the comparison allows for predicting the clinical course of Acute Myelogenous Leukemia (AML).

## Patentansprüche

1. Verfahren zum Einrichten eines Kombinationsmarkerprofils für eine Probe, die von einem Individuum gewonnen wurde, bei dem der Verdacht auf einen neoplastischen Zustand besteht, wobei das Verfahren die folgenden Schritte umfasst:
(a) Umsetzen der biologischen Probe mit einer Kollektion von Bindemolekülen, wobei die Bindemoleküle zwei oder mehr Gruppen von Bindemolekülen aufweisen, die spezifisch sind für mit unterschiedlichen Zellpopulationen assoziierte Marker, wobei die Markerspiegel und die Markerkombinationen normale und neoplastische Zellpopulationen innerhalb der Probe nachweisen,
(b) Nachweisen des Vorhandenseins normaler und neoplastischer Zellpopulationen innerhalb der Probe durch Erfassen der Markerspiegel und Markerkombinationen, die die normalen und neoplastischen Zellpopulationen innerhalb der Probe nachweisen, und
(c) Korrelieren der Markerspiegel über sowohl die normalen als auch die neoplastischen Zellpopulationen mit dem Vorhandensein eines Targetproteins, um ein Kombinationsmarkerprofil für eine Probe einzurichten, die von einem Individuum gewonnen wurde, bei dem der Verdacht auf einen neoplastischen Zustand besteht, wobei das Targetprotein ein Signalmolekül ist.

2. Verfahren nach Anspruch 1, wobei das Targetprotein ein aktivierungsphosphoryliertes Signalübertragungsprotein ist.

3. Verfahren nach Anspruch 1, ferner eine Bestimmung einer Targetproteinmodifikation umfassend.

4. Verfahren nach Anspruch 1, wobei der neoplastische Zustand ausgewählt ist aus der Gruppe bestehend aus chronisch-lymphatischer Leukämie (CLL), akuter myeloischer Leukämie (AML), chronischer myeloischer Leukämie (CML) und akut-lymphatischer Leukämie (ALL).

5. Verfahren nach Anspruch 1, wobei die mit Zellpopulationen assoziierten Marker Zelloberflächenmarker umfassen, die ausgewählt sind aus der Gruppe bestehend aus CD3, CD5, CD10, CD11b, CD13, CD 15, CD14, CD 15, CD16, CD19, CD22, CD 23, CD56, CD45, CD33, CD34, CD15, CD16, MPL (Myeloperoxidase), CD64, CD79a, CD79b und CD117 (c-Kit-rezeptor).

6. Verfahren nach Anspruch 5, wobei die Marker ferner leichte kappa- und lambda-Immunoglobulinketten aufweisen, um eine Klonalität zu bestimmen.

7. Verfahren nach Anspruch 4, wobei der Krebs chronisch-lymphatische B-Zellenleukämie (B-CLL) ist.

8. Verfahren nach Anspruch 7, wobei das Targetprotein ausgewählt ist aus der Gruppe bestehend aus ZAP-70, aktivierungsinduziertem C-Typ-Lectin (AICL), Lipoproteinlipase und IM68532.

9. Verfahren nach Anspruch 8, wobei die chronisch-lymphatische B-Zellen-Leukämie (B-CLL) Ig-mutierte B-CLL ist.

10. Verfahren nach Anspruch 7, wobei die mit Zellpopulationen assoziierten Marker ausgewählt sind aus der Gruppe bestehend aus CD3, CD5, CD 19, CD 23, CD38, CD56, CD 79b und fmc 7.

11. Verfahren nach Anspruch 10, wobei die mit Zellpopulationen assoziierten Marker eine Zellpopulation nachweisen, die leukämische B-Zellen umfasst.

12. Verfahren nach Anspruch 11, wobei die leukämischen B-Zellen ZAP-70-positiv sind.

13. Verfahren nach Anspruch 10, wobei eine oder mehrere der Zellpopulationen ZAP-70-negative Zellpopulationen umfasst bzw. umfassen.

14. Verfahren nach Anspruch 10, wobei eine oder mehrere der Zellpopulationen normale B-Zellen umfasst bzw. umfassen.

15. Verfahren nach Anspruch 13, wobei die ZAP-negative Zellpopulation Granulozyten umfasst.

16. Verfahren zum Vorhersagen des klinischen Verlaufs von Ig-unmutierter chronisch-lymphatischer B-Zellen-Leukämie (CLL) bei einem Individuum, wobei das Verfahren die folgenden Schritte umfasst:
(a) Bereitstellen einer biologischen Probe, die von dem Individuum gewonnen wurde;
(b) Umsetzen der biologischen Probe mit einer Kollektion von Bindemolekülen, wobei die Bindemoleküle zwei oder mehr Gruppen von Bindemolekülen enthalten, die spezifisch sind für mit unterschiedlichen Zellpopulationen assoziierte Marker, wobei die Markerspiegel und Markerkombinationen normale und neoplastische Zellpopulationen innerhalb der Probe nachweisen,
(c) Nachweisen des Vorhandenseins normaler und neoplastischer Zellpopulationen innerhalb der Probe durch Erfassen der Markerspiegel und Markerkombinationen, die die normalen und neoplastischen Zellpopulationen innerhalb der Probe nachweisen,
(d) Korrelieren der Markerspiegel über sowohl die normalen als auch die neoplastischen Zellpopulationen mit dem Vorhandensein von ZAP-70, um ein Kombinationsmarkerprofil für eine Probe einzurichten, die von einem Individuum gewonnen wurde, bei dem der Verdacht auf chronisch-lymphatische B-Zellenleukämie (B-CLL) besteht, wobei das zusammengesetzte Profil eine relative Quantifizierung von ZAP-70-Spiegeln darstellt; und
(e) Vergleichen des Kombinationsprofils mit einem oder mehreren Kombinations-Bezugsprofilen, wobei der Vergleich die Voraussage des klinischen Verlaufs von Ig-unmutierter chronisch-lymphatischer Leukämie (CLL) erlaubt.

17. Verfahren zum Einrichten eines Kombinationsmarkerprofils für eine Probe, die von einem Individuum gewonnen wurde, bei dem der Verdacht auf chronische myeloische Leukämie (CML) besteht, wobei das Verfahren die folgenden Schritte umfasst:
(a) Umsetzen der biologischen Probe mit einer Kollektion von Bindemolekülen, wobei die Kollektion von Bindemolekülen zwei oder mehr Gruppen von Bindemolekülen enthält, die spezifisch sind für mit unterschiedlichen Zellpopulationen assoziierte Marker, wobei die Markerspiegel und Markerkombinationen normale und neoplastische Zellpopulationen innerhalb der Probe nachweisen,
(b) Nachweisen des Vorhandenseins normaler und neoplastischer Zellpopulationen innerhalb der Probe durch Erfassen der Markerspiegel und Markerkombinationen, die die normalen und neoplastischen Zellpopulationen innerhalb der Probe nachweisen, und
(c) Korrelieren der Markerspiegel über sowohl die normalen als auch die neoplastischen Zellpopulationen mit dem Vorhandensein mindestens eines Targetproteins, das ausgewählt ist aus der Gruppe bestehend aus aktivierungsphosphorylierten Signalübertragungsproteinen, Proliferationsmarkern, Differenzierungsmarkem und Apoptosemarkem, um ein Kombinationsmarkerprofil für eine Probe einzurichten, die von einem Individuum gewonnen wurde, bei dem der Verdacht auf chronische myeloische Leukämie (CML) besteht.

18. Verfahren nach Anspruch 17, wobei die mit Zellpopulationen assoziierten Marker ausgewählt sind aus der Gruppe bestehend aus CD45, CD34, CD11b, CD 13, CD 15, CD14, CD33, CD79a, CD79b, CD22, CD10, CD16, Bcr/Abl und TdT.

19. Verfahren nach Anspruch 17, wobei das Targetprotein ein aktivierungsphosphoryliertes Signalübertragungsprotein ist.

20. Verfahren nach Anspruch 19, wobei das aktivierungsphosphorylierte Signalübertragungsprotein ausgewählt ist aus der Gruppe bestehend aus Abl, CRKL, Hck, STAT1, STAT3, STAT5, Akt/PKB und S6.

21. Verfahren nach Anspruch 17, wobei das Targetprotein ein Proliferationsmarker oder ein Apoptosemarker ist.

22. Verfahren nach Anspruch 21, wobei das Targetprotein ein Proliferationsmarker ist, der ausgewählt ist aus der Gruppe bestehend aus Cyclin D1 und Cyclin A2.

23. Verfahren nach Anspruch 21, wobei das Targetprotein ein Apoptosemarker ist, der ausgewählt ist aus der Gruppe bestehend aus Caspase-3 und Bcl-X1.

24. Verfahren zum Vorhersagen des klinischen Verlaufs von chronischer myeloischer Leukämie (CML) in einem Individuum, wobei das Verfahren die folgenden Schritte umfasst:
(a) Bereitstellen einer biologischen Probe, die von dem Individuum gewonnen wurde;
(b) Umsetzen der biologischen Probe mit einer Kollektion von Bindemolekülen, wobei die Kollektion von Bindemolekülen zwei oder mehr Gruppen von Bindemolekülen enthält, die spezifisch sind für mit unterschiedlichen Zellpopulationen assoziierte Marker, wobei die Markerspiegel und Markerkombinationen normale und neoplastische Zellpopulationen innerhalb der Probe nachweisen,
(c) Nachweisen des Vorhandenseins normaler und neoplastischer Zellpopulationen innerhalb der Probe durch Erfassen der Markerspiegel und Markerkombinationen, die die normalen und neoplastischen Zellpopulationen innerhalb der Probe nachweisen,
(d) Korrelieren der Markerspiegel in sowohl den normalen als auch den neoplastischen Zellpopulationen mit dem Vorhandensein mindestens eines Targetproteins, das ausgewählt ist
aus der Gruppe bestehend aus aktivierungsphosphorylierten Signalübertragungsproteinen, Proliferationsmarkem und Apoptosemarkem, um ein Kombinationsmarkerprofil für eine Probe einzurichten, die von einem Individuum gewonnen wurde, bei dem der Verdacht auf chronische myeloische Leukämie (CML) besteht, und
(e) Vergleichen des Kombinationsprofils mit einem oder mehreren Bezugs-Kombinationsprofilen, wobei der Vergleich die Voraussage des klinischen Verlaufs von chronischer myeloischer Leukämie (CML) erlaubt.

25. Verfahren zum Einrichten eines Kombinationsmarkerprofils für eine Probe, die von einem Individuum gewonnen wurde, bei dem der Verdacht auf akute myeloische Leukämie (AML) besteht, wobei das Verfahren die folgenden Schritte umfasst:
(a) Umsetzen der biologischen Probe mit einer Kollektion von Bindemolekülen, wobei die Kollektion von Bindemolekülen zwei oder mehr Gruppen von Bindemolekülen enthält, die spezifisch sind für mit unterschiedlichen Zellpopulationen assoziierte Marker, wobei die Markerspiegel und Markerkombinationen normale und neoplastische Zellpopulationen innerhalb der Probe nachweisen,
(b) Nachweisen des Vorhandenseins normaler und neoplastischer Zellpopulationen innerhalb der Probe durch Erfassen der Markerspiegel und Markerkombinationen, die die normalen und neoplastischen Zellpopulationen innerhalb der Probe nachweisen, und
(c) Korrelieren der Markerspiegel über sowohl die normalen als auch die neoplastischen Zellpopulationen mit dem Vorhandensein mindestens eines Targetproteins, das ausgewählt ist aus der Gruppe bestehend aus aktivierungsphosphorylierten Signalübertragungsproteinen, Proliferationsmarkem und Apoptosemarkem, um ein Kombinationsmarkerprofil für eine Probe einzurichten, die von einem Individuum gewonnen wurde, bei dem der Verdacht auf akute myeloische Leukämie (AML) besteht.

26. Verfahren nach Anspruch 25, wobei die mit einer Zellpopulation assoziierten Marker ausgewählt sind aus der Gruppe bestehend aus CD45, CD33, CD34, CD11b, CD13, CD14, CD 15, CD16, MPL (Myeloperoxidase), CD64 und CD117 (c-Kitrezeptor).

27. Verfahren nach Anspruch 26, wobei die mit Zellpopulationen assoziierten Marker eine Zellpopulation nachweisen, die leukämische Granulozyten oder leukämische Monozyten umfasst.

28. Verfahren nach Anspruch 25, wobei das Targetprotein ein aktivierungsphosphoryliertes Signalübertragungsprotein ist.

29. Verfahren nach Anspruch 25, wobei das aktivierungsphosphorylierte Signalübertragungsprotein ausgewählt ist aus der Gruppe bestehend aus Abl, CRKL, Hck, STAT1, STAT3, STAT5, Akt/PKB und S6.

30. Verfahren nach Anspruch 25, wobei das Targetprotein ein Proliferationsmarker oder ein Apoptosemarker ist.

31. Verfahren nach Anspruch 30, wobei das Zielprotein ein Proliferationsmarker ist, der ausgewählt ist aus der Gruppe bestehend aus Cyclin D1 und Cyclin A2.

32. Verfahren nach Anspruch 30, wobei das Targetprotein ein Apoptosemarker ist, der ausgewählt ist aus der Gruppe bestehend aus Caspase-3 und Bcl-X1.

33. Verfahren zum Vorhersagen des klinischen Verlaufs von akuter myeloischer Leukämie (AML) in einem Individuum, wobei das Verfahren die folgenden Schritte umfasst:
(b) Umsetzen der biologischen Probe, die von dem Individuum erhalten wurde, mit einer Kollektion von Bindemolekülen, wobei die Bindemoleküle zwei oder mehr Gruppen von Bindemolekülen enthalten, die spezifisch sind für mit unterschiedlichen Zellpopulationen assoziierte Marker, wobei die Markerspiegel und Markerkombinationen normale und neoplastische Zellpopulationen innerhalb der Probe nachweisen,
(c) Nachweisen des Vorhandenseins normaler und neoplastischer Zellpopulationen innerhalb der Probe durch Erfassen der Markerspiegel und Markerkombinationen, die die normalen und neoplastischen Zellpopulationen innerhalb der Probe nachweisen,
(d) Korrelieren der Markerspiegel über sowohl die normalen als auch die neoplastischen Zellpopulationen mit dem Vorhandensein mindestens eines Targetproteins, das ausgewählt ist aus der Gruppe bestehend aus aktivierungsphosphorylierten Signalübertragungsproteinen, Proliferationsmarkem und Apoptosemarkem, um ein Kombinationsmarkerprofil für eine Probe einzurichten, die von einem Individuum gewonnen wurde, bei dem der Verdacht auf akute myeloische Leukämie (AML) besteht; und
(e) Vergleichen des zusammengesetzten Profils mit einem oder mehreren Bezugs-Kombinationsprofilen, wobei der Vergleich die Voraussage des klinischen Verlaufs von akuter myeloischer Leukämie (AML) erlaubt.

## Revendications

1. Procédé permettant d'établir un profil de marqueur composite pour un échantillon prélevé sur un sujet suspecté d'être atteint d'un trouble néoplasique, le procédé comprenant les étapes consistant à :
(a) faire réagir l'échantillon biologique avec un ensemble de molécules de liaison, l'ensemble de molécules de liaison contenant deux groupes ou plus de molécules de liaison spécifiques de marqueurs associés à une population cellulaire distincte, les concentrations en marqueurs et les combinaisons de marqueurs identifiant les populations cellulaires normales et néoplasiques internes à l'échantillon,
(b) identifier la présence de populations cellulaires normales et néoplasiques internes à l'échantillon en détectant les concentrations en marqueurs et les combinaisons de marqueurs qui identifient les populations cellulaires normales et néoplasiques internes à l'échantillon, et
(c) corréler les concentrations en marqueurs de chacune des populations cellulaires normales et néoplasiques avec la présence d'une protéine cible pour établir un profil de marqueur composite pour un échantillon prélevé sur un sujet suspecté d'être atteint d'un trouble néoplasique, la protéine cible étant une molécule de signalisation.

2. Procédé selon la revendication 1, dans lequel ladite protéine cible est une protéine de transduction de signal phosphorylée par activation.

3. Procédé selon la revendication 1, comprenant en outre une détermination de modification de la protéine cible.

4. Procédé selon la revendication 1, dans lequel le trouble néoplasique est choisi dans le groupe constitué de la leucémie lymphoïde chronique (LLC), la leucémie myéloïde aiguë (LMA), la leucémie myéloïde chronique (LMC), et la leucémie lymphoïde aiguë (LLA).

5. Procédé selon la revendication 1, dans lequel les marqueurs associés à la population cellulaire comprennent des marqueurs de surface des cellules choisis dans le groupe constitué de CD3, CD5, CD10, CD11b, CD13, CD15, CD14, CD15, CD16, CD19, CD22, CD23, CD56, CD45, CD33, CD34, CD15, CD16, MPL (myéloperoxydase), CD64, CD79a, CD79b, et CD117 (récepteur c-kit).

6. Procédé selon la revendication 5, dans lequel les marqueurs incluent en outre des chaînes légères d'immunoglobines kappa et lambda pour déterminer la clonalité.

7. Procédé selon la revendication 4, dans lequel ledit cancer est la leucémie lymphoïde chronique B (LLC-B).

8. Procédé selon la revendication 7, dans lequel ladite protéine cible est choisie dans le groupe constitué de ZAP-70, la lectine de type C induite par activation (LCIA), la lipoprotéine lipase et IM68532.

9. Procédé selon la revendication 8, dans lequel la leucémie lymphoïde chronique B (LLC-B) est LLC-B à Ig mutées.

10. Procédé selon la revendication 7, dans lequel les marqueurs associés à la population cellulaire sont choisis dans le groupe constitué de CD3, CD5, CD19, CD23, CD38, CD56, CD79b et fmc 7.

11. Procédé selon la revendication 10, dans lequel les marqueurs associés à la population cellulaire identifient une population cellulaire comprenant des cellules B leucémiques.

12. Procédé selon la revendication 11, dans lequel les cellules B leucémiques sont ZAP-70 positives.

13. Procédé selon la revendication 10, dans lequel une ou plusieurs populations cellulaires comprennent des populations cellulaires ZAP-70 négatives.

14. Procédé selon la revendication 10, dans lequel une ou plusieurs populations cellulaires comprennent des cellules B normales.

15. Procédé selon la revendication 13, dans lequel la population cellulaire ZAP-70 négative comprend des granulocytes.

16. Procédé permettant de prédire l'évolution clinique de la leucémie lymphoïde chronique B à Ig non mutées (LLC) chez un sujet, le procédé comprenant les étapes consistant à :
(a) fournir un échantillon biologique provenant du sujet ;
(b) faire réagir l'échantillon biologique avec un ensemble de molécules de liaison, l'ensemble de molécules de liaison contenant deux groupes ou plus de molécules de liaison spécifiques de marqueurs associés à une population cellulaire distincte, les concentrations en marqueurs et les combinaisons de marqueurs identifiant les populations cellulaires normales et néoplasiques internes à l'échantillon,
(c) identifier la présence de populations cellulaires normales et néoplasiques internes à l'échantillon en détectant les concentrations en marqueurs et les combinaisons de marqueurs qui identifient les populations cellulaires normales et néoplasiques internes à l'échantillon,
(d) corréler les concentrations en marqueurs de chacune des populations cellulaires normales et néoplasiques avec la présence de ZAP-70 pour établir un profil de marqueur composite pour un échantillon prélevé sur un sujet suspecté d'être atteint de leucémie lymphoïde chronique B (LLC-B), le profil composite représentant une quantification relative des concentrations en ZAP-70; et
(e) comparer le profil composite avec un ou plusieurs profils composites de référence, la comparaison permettant de prédire l'évolution clinique de la leucémie lymphoïde chronique B à Ig non mutées (LLC).

17. Procédé permettant d'établir le profil de marqueur composite pour un échantillon prélevé sur un sujet suspecté d'être atteint de leucémie myéloïde chronique (LMC), le procédé comprenant les étapes consistant à :
(a) faire réagir l'échantillon biologique avec un ensemble de molécules de liaison, l'ensemble de molécules de liaison contenant deux groupes ou plus de molécules de liaison spécifiques de marqueurs associés à une population cellulaire distincte, les concentrations en marqueurs et les combinaisons de marqueurs identifiant les populations cellulaires normales et néoplasiques internes à l'échantillon,
(b) identifier la présence de populations cellulaires normales et néoplasiques internes à l'échantillon en détectant les concentrations en marqueurs et les combinaisons de marqueurs qui identifient les populations cellulaires normales et néoplasiques internes à l'échantillon, et
(c) corréler les concentrations en marqueurs de chacune des populations cellulaires normales et néoplasiques avec la présence d'au moins une protéine cible choisie dans le groupe constitué de protéines de transduction de signal phosphorylées par activation, de marqueurs de prolifération, de marqueurs de différentiation et de marqueurs d'apoptose pour établir un profil de marqueur composite pour un échantillon prélevé sur un sujet suspecté d'être atteint de leucémie myéloïde chronique (LMC).

18. Procédé selon la revendication 17, dans lequel les marqueurs associés à la population cellulaire sont choisis dans le groupe constitué de CD45, CD34, CD11b, CD13, CD15, CD14, CD33, CD79a, CD79b, CD22, CD10, CD16, Bcr/Abl et TdT.

19. Procédé selon la revendication 17, la protéine cible est une protéine de transduction de signal phosphorylée par activation.

20. Procédé selon la revendication 19, la protéine de transduction de signal phosphorylée par activation est choisie dans le groupe constitué de Abl, CRKL, Hck, STAT1, STAT3, STAT5, Akt/PKB et S6.

21. Procédé selon la revendication 17, la protéine cible est un marqueur de prolifération ou un marqueur d'apoptose.

22. Procédé selon la revendication 21, dans lequel la protéine cible est un marqueur de prolifération qui est choisi dans le groupe constitué de la Cycline D1 et la Cycline A2.

23. Procédé selon la revendication 21, dans lequel la protéine cible est un marqueur d'apoptose choisi dans le groupe constitué de la Caspase-3 et de Bcl-X1.

24. Procédé permettant de prédire l'évolution clinique de la leucémie myéloïde chronique (LMC) chez un sujet, le procédé comprenant les étapes consistant à :
(a) fournir un échantillon biologique prélevé sur un sujet ;
(b) faire réagir l'échantillon biologique avec un ensemble de molécules de liaison, l'ensemble de molécules de liaison contenant deux groupes ou plus de molécules de liaison spécifiques de marqueurs associés à une population cellulaire distincte, les concentrations en marqueurs et les combinaisons de marqueurs identifiant les populations cellulaires normales et néoplasiques internes à l'échantillon,
(c) identifier la présence de populations cellulaires normales et néoplasiques internes à l'échantillon en détectant les concentrations en marqueurs et les combinaisons de marqueurs qui identifient les populations cellulaires normales et néoplasiques internes à l'échantillon,
(d) corréler les concentrations en marqueurs de chacune des populations cellulaires normales et néoplasiques avec la présence d'au moins une protéine cible choisie dans le groupe constitué de protéines de transduction de signal phosphorylées par activation, de marqueurs de prolifération et de marqueurs d'apoptose pour établir un profil de marqueur composite pour un échantillon prélevé sur un sujet suspecté d'être atteint de leucémie myéloïde chronique (LMC), et
(e) comparer le profil composite avec un ou plusieurs profils composites de référence, la comparaison permettant de prédire l'évolution clinique de la leucémie myéloïde chronique (LMC).

25. Procédé permettant d'établir le profil de marqueur composite pour un échantillon prélevé sur un sujet suspecté d'être atteint de leucémie myéloïde aiguë (LMA), le procédé comprenant les étapes consistant à :
(a) faire réagir l'échantillon biologique avec un ensemble de molécules de liaison, l'ensemble de molécules de liaison contenant deux groupes ou plus de molécules de liaison spécifiques de marqueurs associés à une population cellulaire distincte, les concentrations en marqueurs et les combinaisons de marqueurs identifiant les populations cellulaires normales et néoplasiques internes à l'échantillon,
(b) identifier la présence de populations cellulaires normales et néoplasiques internes à l'échantillon en détectant les concentrations en marqueurs et les combinaisons de marqueurs qui identifient les populations cellulaires normales et néoplasiques internes à l'échantillon, et
(c) corréler les concentrations en marqueurs de chacune des populations cellulaires normales et néoplasiques avec la présence d'au moins une protéine cible choisie dans le groupe constitué de protéines de transduction de signal phosphorylées par activation, de marqueurs de prolifération et de marqueurs d'apoptose pour établir un profil de marqueur composite pour un échantillon prélevé sur un sujet suspecté d'être atteint de leucémie myéloïde aiguë (LMA).

26. Procédé selon la revendication 25, dans lequel les marqueurs associés à la population cellulaire sont choisis dans le groupe constitué de CD45, CD33, CD34, CD11b, CD13, CD 14, CD 15, CD16, MPL (myéloperoxydase), CD64 et CD 117 (récepteur c-kit).

27. Procédé selon la revendication 26, dans lequel les marqueurs associés à la population cellulaire identifient une population cellulaire comprenant des granulocytes leucémiques ou des monocytes leucémiques.

28. Procédé selon la revendication 25, dans lequel la protéine cible est une protéine de transduction de signal phosphorylée par activation.

29. Procédé selon la revendication 25, dans lequel la protéine de transduction de signal phosphorylée par activation est choisie dans le groupe constitué de Abl, CRKL, Hck, STAT1, STAT3, STAT5, Akt/PKB et S6.

30. Procédé selon la revendication 25, dans lequel la protéine cible est un marqueur de prolifération ou un marqueur d'apoptose.

31. Procédé selon la revendication 30, dans lequel la protéine cible est un marqueur de prolifération choisi dans le groupe constitué de la Cycline D1 et la Cycline A2.

32. Procédé selon la revendication 30, dans lequel la protéine cible est un marqueur d'apoptose choisi dans le groupe constitué de la Caspase-3 et de Bcl-X1.

33. Procédé permettant de prédire l'évolution clinique de la leucémie myéloïde aiguë (LMA) chez un sujet, le procédé comprenant les étapes consistant à :
(b) faire réagir l'échantillon biologique prélevé sur l'sujet avec un ensemble de molécules de liaison, étape dans laquelle l'ensemble de molécules de liaison contient deux groupes ou plus de molécules de liaison spécifiques de marqueurs associés à une population cellulaire distincte, les concentrations en marqueurs et les combinaisons de marqueurs identifiant les populations cellulaires normales et néoplasiques internes à l'échantillon,
(c) identifier la présence de populations cellulaires normales et néoplasiques internes à l'échantillon en détectant les concentrations en marqueurs et les combinaisons de marqueurs qui identifient les populations cellulaires normales et néoplasiques internes à l'échantillon,
(d) corréler les concentrations en marqueurs de chacune des populations cellulaires normales et néoplasiques avec la présence d'au moins une protéine cible choisie dans le groupe constitué de protéines de transduction de signal phosphorylées par activation, de marqueurs de prolifération et de marqueurs d'apoptose pour établir un profil de marqueur composite pour un échantillon prélevé sur un sujet suspecté d'être atteint de leucémie myéloïde aiguë (LMA) ; et
(e) comparer le profil composite avec un ou plusieurs profils composites de référence, la comparaison permettant de prédire l'évolution clinique de la leucémie myéloïde aiguë (LMA).
